(19) Europäisches Patentamt
European Patent Office
Office européen des brevets

(11) Publication number: 0 347 216 B1

(12) # EUROPEAN PATENT SPECIFICATION

(45) Date of publication of patent specification: **01.09.93**

(51) Int. Cl.[5]: **C07C 241/00**, C07C 241/04, C07C 257/22, C07C 243/00, C07C 327/00, A01N 33/26, A01N 37/28

(21) Application number: **89306052.5**

(22) Date of filing: **15.06.89**

(54) **Insecticidal N'-substituted-N-alkylcarbonyl-N'-acylhydrazines and N'-substituted -N-acyl-N'-alkylcarbonyl hydrazines.**

(30) Priority: **15.06.88 US 207081**

(43) Date of publication of application:
**20.12.89 Bulletin 89/51**

(45) Publication of the grant of the patent:
**01.09.93 Bulletin 93/35**

(84) Designated Contracting States:
**AT BE CH DE ES FR GB GR IT LI LU NL SE**

(56) References cited:
**EP-A- 0 232 075**
**EP-A- 0 234 944**
**EP-A- 0 236 618**

(73) Proprietor: **ROHM AND HAAS COMPANY**
**Independence Mall West**
**Philadelphia Pennsylvania 19105(US)**

(72) Inventor: **Hsu, Adam Chi-Tung**
**1686 Heebner Way**
**Lansdale Pennsylvania 19446(US)**
Inventor: **Le, Dat Phat**
**125 Washington Avenue**
**North Wales Pennsylvania 19454(US)**
Inventor: **Murphy, Raymond August**
**Forge Gate Apartments Apt. 38-B-1**
**Lansdale Pennsylvania 19446(US)**
Inventor: **Weinstein, Barry**
**419 Bluebird Lane**
**Dresher Pennsylvania 19025(US)**

(74) Representative: **Harding, Charles Thomas et al**
**ROHM AND HAAS (UK) LTD., European Operations Patent Dept., Lennig House, 2 Mason's Avenue**
**Croydon CR9 3NB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention concerns novel N′-substituted-N-alkylcarbonyl-N′-acylhydrazines or N′-substituted-N-acyl-N′-alkylcarbonylhydrazineswhich are useful as insecticides, compositions containing those compounds and methods of their use.

The search for compounds which have a combination of excellent insecticidal activity and low undesirable toxicity is a continuing one because of factors such as the desire for compounds exhibiting greater activity, better selectivity, low undesirable environmental impact, low production cost and effectiveness against insects resistant to many known insecticides.

Compounds of the present invention are particularly suitable for controlling plant-destructive insects in crops of cultivated plants, ornamentals and forestry.

Certain hydrazine and/or hydrazide derivatives have been disclosed in the literature.

U.S. Patent No. 3,481,972 discloses 2-(beta-hydroxyethyl)-2-methyl acid hydrazine compounds of the formula $ZC(O)NHN(CH_3)CH_2CH_2OH$ where Z represents furyl, cyclohexyl, phenyl, benzyl, styryl, dihydrostyryl or substituted phenyl. The compounds are stated to be useful as pesticides for the control of such organisms as insects, arachnids, nematodes, fungi, plants and helminth organisms.

U.S. Patent No. 4,564,611 discloses (di)thiophosphoric and -phosphonic acid derivatives having the formula

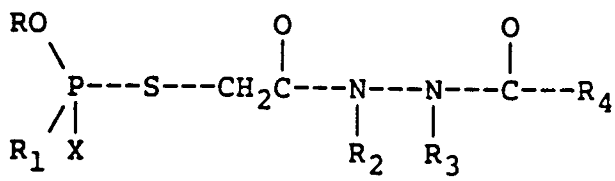

in which R denotes $(C_1\text{-}C_4)$alkyl; $R_1$ denotes $(C_1\text{-}C_4)$alkyl, $(C_1\text{-}C_4)$alkoxy, $(C_1\text{-}C_4)$alkylmercapto, $(C_1\text{-}C_4)$-alkylamino or di$(C_1\text{-}C_4)$alkylamino; $R_2$ and $R_3$ independently of one another denote hydrogen, $(C_1\text{-}C_4)$alkyl, $(C_5\text{-}C_6)$cycloalkyl, benzyl or furylmethyl; $R_4$ denotes $(C_1\text{-}C_4)$alkyl, $(C_1\text{-}C_3)$alkoxymethyl, $(C_1\text{-}C_3)$-alkylmercaptomethyl or phenyl and X denotes oxygen or sulfur, which have activity against sucking and biting insects, acarides and nematodes, and display good fungicidal activity. Insect growth regulating activity is not disclosed. Further, $R_2$ is not taught to include a tertiary carbon.

In 88 J.A.C.S, 4677-4681 (1966) N′-t-butyl-N′-methylcarbonyl-N-benzoylhydrazine is disclosed. No biological activity is disclosed.

In 25 Aust. J. Chem., 523-529 (1972), several N,N′-dibenzoylhydrazine derivatives are disclosed in which one or both nitrogen atoms are alkylated or phenylated. No biological activity is disclosed for those compounds.

In 61 Helv. Chim. Acta, 1477-1510 (1978), several N,N′-dibenzoylhydrazine and hydrazide derivatives are disclosed. No biological activity is disclosed for those compounds.

In 44 J.A.C.S., 2556-2567 (1922), isopropyl hydrazine $(CH_3)_2CH\text{-}NH\text{-}NH_2$, symmetrical diisopropyl hydrazine, dibenzoylisopropyl hydrazine and certain derivatives are disclosed. No biological activity is disclosed for those compounds.

In 44 J.A.C.S., 1557-1564 (1972), isopropyl, menthyl and bornyl semicarbazides are disclosed. No biological activity is disclosed for those compounds.

In 48 J.A.C.S., 1030-1035 (1926), symmetrical di-methylphenylmethyl hydrazine and certain related compounds including 1,2-bis-methylphenylmethyl-4-phenylsemicarbazide are disclosed. No biological activity is disclosed for those compounds.

In 27 Bull. Chem. Soc. Japan, 624-627 (1954), certain hydrazine derivatives including alpha,beta-dibenzoylphenyl hydrazine are disclosed. No biological activity is disclosed for those compounds.

In J. Chem. Soc. (C), 1531-1536 (1966), N,N′-dibenzoylphenyl hydrazine and N-acetyl-N′-benzoyl-p-nitrophenyl hydrazine are disclosed. No biological activity is disclosed for those compounds.

In 56B Chem. Berichte, 954-962 (1923), symmetrical di-isopropyl hydrazines, symmetrical diisobutyl and certain derivatives including N,N′-diisobutyldibenzoyl hydrazine are disclosed. No biological activity is disclosed for those compounds.

In 590 Annalen der Chemie, 1-36 (1954), certain N,N′-dibenzoyl hydrazine derivatives are disclosed including N′-methyl- and N′-(2-phenyl)-isopropyl-N,N′-dibenzoyl hydrazine. No biological activity is disclosed for those compounds.

In J. Chem. Soc., 4191-4198 (1952), N,N'-di-n-propyl hydrazine and the bis-3,5-dinitrobenzoyl derivatives are disclosed. No biological activity is disclosed for those compounds.

In 32 Zhur. Obs. Khim., 2806-2809 (1962), N'-2,4-methyl-2,4-pentadiene-N,N'-dibenzoyl hydrazine is disclosed. No biological activity is disclosed.

In 17 Acta. Chim. Scand., 95-102 (1963), 2-benzoylthiobenzhydrazide ($C_6H_5$-CS-NHNH-CO-$C_6H_5$) and certain hydrazone and hydrazine derivatives are disclosed including 1,2-dibenzoyl-benzyl hydrazine. No biological activity is disclosed for those compounds.

In 25 Zhur. Obs. Khim, 1719-1723 (1955), N,N'-bis-cyclohexyl hydrazine and N,N'-dibenzoylcyclohexyl hydrazine are disclosed. No biological activity is disclosed for those compounds.

In J. Chem. Soc., 4793-4800 (1964), certain dibenzoyl hydrazine derivatives are disclosed including tribenzoyl hydrazine and N,N'-dibenzoylcyclohexyl hydrazine. No biological activity is disclosed for those compounds.

In 36 J. Prakt. Chem., 197-201 (1967), certain dibenzoyl hydrazine derivatives including N'-ethyl-; N'-n-propyl-; N'-isobutyl-; N-neopentyl-; N'-n-heptyl-; and N'-cyclohexylmethyl-N,N'-dibenzoyl hydrazines are disclosed. No biological activity is disclosed for those compounds.

In 26 J.O.C., 4336-4340 (1961) N'-t-butyl-N,N'-di-(t-butoxycarbonyl) hydrazide is disclosed. No biological activity is disclosed.

In 41 J.O.C., 3763-3765 (1976), N'-t-butyl-N-(phenylmethoxycarbonyl)-N'-(chlorocarbonyl)hydrazide is disclosed. No biological activity is disclosed.

In 94 J.A.C.S., 7406-7416 (1972) N'-t-butyl-N,N'-dimethoxycarbonyl hydrazide is disclosed. No biological activity is disclosed.

In 43 J.O.C., 808-815 (1978), N'-t-butyl-N-ethoxycarbonyl-N'-phenylaminocarbonyl-hydrazide and N'-t-butyl-N-ethoxycarbonyl-N'-methylaminocarbonyl hydrazide are disclosed. No biological activity is disclosed for those compounds.

In 26 J.A.C., 4336-4340 (1961), N't-butyl-N,N'-di-(t-butoxycarbonyl)hydrazide is disclosed. No biological ectivity is disclosed.

In 41 J.O.C., 3763-3765 (1976), N'-t-butyl-N-(phenylmethoxycarbonyl)-N'-(chlorocarbonyl)hydrazide is disclosed. No biological activity is disclosed.

In 94 J.A.C.S., 7406-7416 (1972), N'-t-butyl-N,N'-dimethoxycarbonyl hydrazide is disclosed. No biological activity is disclosed.

In 39 J. Econ. Ent., 416-417 (1946), certain N-phenyl-N'-acylhydrazines are disclosed and evaluated for their toxicity against codling moth larvae.

The N'-substituted-N-alkylcarbonyl-N'-acylhydrazines of the present invention differ from known compounds primarily by their N- and N'-substituents.

Compounds of the present invention are also distinguished by their excellent insecticidal activity against insects of the orders Lepidoptera and Coleoptera.

In accordance with the present invention, there are provided insecticidal compositions and methods of using such compositions wherein the compositions comprise an agronomically acceptable carrier and an insecticidally effective amount of a compound having the formula:

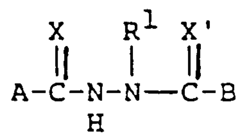

I

wherein

| | |
|---|---|
| X and X' | are the same or different O, S or NR; |
| $R^1$ is | unsubstituted ($C_3$-$C_{10}$) branched alkyl or a ($C_1$-$C_4$) straight chain alkyl substituted with one or two of the same or different ($C_3$-$C_6$)cycloalkyl; and |
| one of A and B is | unsubstituted naphthyl or substituted naphthyl where the substituents can be from one to three of the same or different halo; cyano; nitro; hydroxy; ($C_1$-$C_4$)alkoxy; ($C_1$-$C_4$)alkyl; carboxy; ($C_1$-$C_4$)alkoxycarbonyl; ($C_1$-$C_4$)-alkanoyloxy; amino; ($C_1$-$C_4$)alkylamino or ($C_1$-$C_4$)dialkylamino having independently the stated number of carbon atoms in each alkyl group; or unsubstituted phenyl or substituted phenyl where the substituents can be from one to five of the same or different halo; nitro; cyano; hydroxy; ($C_1$- |

$C_6$)alkyl; ($C_1$-$C_6$)haloalkyl; ($C_1$-$C_6$)cyanoalkyl; ($C_1$-$C_6$)alkoxy; ($C_1$-$C_6$)-haloalkoxy; ($C_1$-$C_6$)alkoxyalkyl having independently the stated number of carbon atoms in each alkyl group; ($C_1$-$C_6$)alkoxyalkoxy having independently the stated number of carbon atoms in each alkyl group; carboxyoxy; ($C_1$-$C_6$)alkoxycarbonyloxy-($C_2$-$C_6$)alkenyl optionally substituted with halo, cyano, ($C_1$-$C_4$)alkyl, ($C_1$-$C_4$)alkoxy, ($C_1$-$C_4$)haloalkoxy or ($C_1$-$C_4$)-alkylthio; carboxy; ($C_1$-$C_6$)carboxyalkyl; ($C_1$-$C_6$)alkoxycarbonylalkyl having independently the stated number of carbon atoms in each alkyl group; -COR; ($C_1$-$C_6$)haloalkylcarbonyl; ($C_1$-$C_6$)cyanoalkylcarbonyl; ($C_1$-$C_6$)-nitroalkylcarbonyl; ($C_1$-$C_6$)alkoxycarbonyl; ($C_1$-$C_6$)haloalkoxycarbonyl; alkanoyloxy; amino; ($C_1$-$C_6$)alkylamino; ($C_1$-$C_6$)dialkylamino having independently the stated number of carbon atoms in each alkyl group; amino or ($C_1$-$C_6$)alkylamino where the N of the amino or ($C_1$-$C_6$)alkylamino is substituted with a hydroxy, ($C_1$-$C_4$)alkoxy or ($C_1$-$C_4$)alkylthio group; phenylamino; diphenylamino; -CONRR'; -OCONRR'; -NRCOR'; -$NRCO_2$R'; -N(COR)COR'; -OCONRCOR'; sulfhydryl; halothio; ($C_1$-$C_6$)-alkylthio; ($C_1$-$C_6$)haloalkylthio; ($C_1$-$C_6$)alkylsulfinyl; ($C_1$-$C_6$)alkylsulfonyl; phenylsulfonyl; ($C_1$-$C_6$)alkylsulfonyloxy;($C_1$-$C_6$)haloalkylsulfonyloxy; -$SO_2$NRR'; -NRSOR'; -$NRSO_2$R'; -CSR; -$CS_2$R; -NRCSR'; -SCOR; unsubstituted phenyl; phenyl substituted with one to three of the same or different halo, cyano, nitro, hydroxy ($C_1$-$C_4$)alkyl, ($C_1$-$C_4$)alkoxy, carboxy, ($C_1$-$C_4$)alkoxycarbonyl, ($C_1$-$C_4$)alkanoyloxy, amino, ($C_1$-$C_4$)alkylamino or ($C_1$-$C_4$)dialkylamino having independently the stated number of carbon atoms in each alkyl group; phenoxy where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)-alkoxycarbonyl, ($C_1$-$C_4$)alkanoyloxy, amino, ($C_1$-$C_4$)alkylamino or ($C_1$-$C_4$)-dialkylamino having independently the stated number of carbon atoms in each alkyl group; or phenylthio where the phenyl ring is unsubstituted or substituted with one to three of the same or different halo, cyano, nitro, hydroxy, ($C_1$-$C_4$)alkyl, ($C_1$-$C_4$)alkoxy, carboxy, ($C_1$-$C_4$)alkoxycarbonyl, ( $C_1$-$C_4$)alkanoyloxy, amino, ($C_1$-$C_4$)alkylamino or ($C_1$-$C_4$)dialkylamino having independently the stated number of carbon atoms in each alkyl group; or when two adjacent positions on the phenyl ring are substituted with alkoxy groups, these groups may be joined to form a 5- or 6-membered dioxolano or dioxano heterocyclic ring; and

the other one of A and B is unsubstituted adamantyl or substituted adamantyl having one to four of the same or different halo, cyano, nitro, hydroxy, ($C_1$-$C_4$)alkyl, halo($C_1$-$C_4$)alkyl, ($C_1$-$C_4$)alkoxy, halo-($C_1$-$C_4$)alkoxy, carboxy, ($C_1$-$C_4$)alkanoyl, ($C_1$-$C_4$)alkoxycarbonyl, ($C_1$-$C_4$)alkanoyloxy, amino, ($C_1$-$C_4$)alkylamino or di-($C_1$-$C_4$)alkylamino;

where

R and R' are independently hydrogen or ($C_1$-$C_6$)alkyl;

and agronomically acceptable salts thereof.

Also, in accordance with the present invention, there are provided insecticidal compounds of Formula I as described and defined above, provided that when $R^1$ is ($C_3$-$C_{10}$)alkyl the alkyl group includes a tertiary carbon atom.

Further, in accordance with the present invention, there are provided methods of using these compounds and compositions.

The term "halo" should be understood as including chloro, fluoro, bromo and iodo. The term "alkyl" by itself or as a part of another substituent, unless otherwise stated, includes straight or branched chain groups such as methyl, ethyl, n-propyl, isopropyl, n-butyl, t-butyl, isobutyl, neopentyl and the like and where indicated higher homologues and isomers such as n-octyl, isooctyl and the like. The term "haloalkyl" by itself or as part of another substituent is an alkyl group of the stated number of carbon atoms having one or more halo atoms bonded thereto such as chloromethyl, 1- or 2-bromoethyl, trifluoromethyl and the like. Analogously, "cyanoalkyl" by itself or as part of another group is an alkyl group of the stated number of carbon atoms having one or more cyano groups bonded thereto; "haloalkoxy" by itself or as part of another group if an alkoxy group of the stated number of carbon atoms having one or more halo atoms bonded

thereto such as difluoromethoxy, trifluoromethoxy, 2-fluoroethoxy, 2,2,2-trifluoroethoxy and the like. "Alkenyl" and "alkynyl" by themselves or as part of another substituent comprise straight and branched chain groups of the stated number of carbon atoms. "Alkadienyl" is a straight or branched chain alkenyl group comprising two carbon-carbon double bonds that can be conjugated such as 1,3-butadienyl, cumulated such as 1,2-propadienyl or isolated such as 1,4-pentadienyl.

Because of their good insecticidal activity, compounds of the present invention for use in the insecticidal compositions and formulations include those where, independently

X and X' are O or S;
$R^1$ is unsubstituted ($C_3$-$C_{10}$) branched alkyl or a ($C_1$-$C_4$) straight chain alkyl substituted with one or two of the same or different ($C_3$-$C_4$)cycloalkyl;
A is adamantyl; and
B is unsubstituted naphthyl; or
unsubstituted phenyl or substituted phenyl where the substituents can be from one to three of the same or different halo; nitro; cyano; ($C_1$-$C_4$)alkyl; ($C_1$-$C_4$)haloalkyl; ($C_1$-$C_4$)cyanoalkyl; ($C_1$-$C_4$)alkoxy; ($C_1$-$C_4$)alkoxyalkyl having independently the stated number of carbon atoms in each alkyl group; -COZ;carboxy; ($C_1$-$C_4$)alkoxycarbonyl; ($C_1$-$C_4$)alkanoyloxy; amino; ($C_1$-$C_4$)alkylamino; ($C_1$-$C_4$)dialkylamino having independently the stated number of carbon atoms in each alkyl group; ($C_1$-$C_4$)alkylthio; -CSZ; -$CS_2$Z; -SCOZ; unsubstituted phenyl; substituted phenyl having one to two of the same or different halo, nitro, ($C_1$-$C_4$)alkyl, ($C_1$-$C_4$)alkoxy, carboxy, ($C_1$-$C_4$)alkoxycarbonyl, ($C_1$-$C_4$)alkanoyloxy, amino; ($C_1$-$C_4$)alkylamino or ($C_1$-$C_4$)dialkylamino having independently the stated number of carbon atoms in each alkyl group or phenoxy where the phenyl ring is unsubstituted or substituted with one or two of the same or different halo, nitro, ($C_1$-$C_4$)alkyl, ($C_1$-$C_4$)alkoxy, carboxy, ($C_1$-$C_4$)-alkoxycarbonyl, ($C_1$-$C_4$)alkanoyloxy, amino, ($C_1$-$C_4$)alkylamino or ($C_1$-$C_4$)dialkylamino having independently the stated number of carbon atoms in each alkyl group; or when two adjacent positions on the phenyl ring are substituted with alkoxy groups, these groups may be joined to form a 5- or 6-membered dioxolano or dioxano heterocyclic ring;
where
Z is hydrogen or ($C_1$-$C_4$)alkyl; and
agronomically acceptable salts thereof.

Insecticidal compounds of the present invention having very good activity for use in the insecticidal compositions and formulations of the present invention include those where, independently,

X and X' are O or S;
$R^1$ is branched ($C_3$-$C_8$)alkyl;
A is adamantyl; and
B is unsubstituted naphthyl; or unsubstituted phenyl or substituted phenyl having one to three of the same or different halo; nitro; cyano; ($C_1$-$C_4$)alkyl; ($C_1$-$C_4$)haloalkyl; ($C_1$-$C_4$)cyanoalkyl; ($C_1$-$C_4$)alkoxy; -COZ; ($C_1$-$C_4$)alkoxycarbonyl; ($C_1$-$C_4$)alkanoyloxy; unsubstituted phenyl or substituted phenyl having one or two of the same or different halo, nitro, ($C_1$-$C_4$)alkyl, ($C_1$-$C_4$)alkoxy, carboxy, ($C_1$-$C_4$)alkoxycarbonyl, ($C_1$-$C_4$)alkanoyloxy, amino; ($C_1$-$C_4$)alkylamino or ($C_1$-$C_4$)dialkylamino having independently the stated number of carbon atoms in each alkyl group; and
where
Z is hydrogen or ($C_1$-$C_4$)alkyl; and
agronomically acceptable salts thereof.

Because of their outstanding insecticidal activity, particularly preferred compounds of the present invention for use in the insecticidal compositions and formulations of the present invention include those where, independently,

X and X' are O;
$R^1$ is t-butyl, 2,2-dimethylpropyl or 1,2,2-trimethylpropyl;
A is adamantyl; and
B is unsubstituted phenyl or
substituted phenyl where the substituents can be one or two of the same or different chloro, fluoro, bromo, iodo, methyl, ethyl, methoxy or trifluoromethyl; and
agronomically acceptable salts thereof.

Those hydrazines of Formula I which possess acidic or basic functional groups may be further reacted to form novel salts with appropriate bases or acids. These salts also exhibit insecticidal activity. Typical salts are the agronomically acceptable metal salts, ammonium salts and acid addition salts. Among the

metal salts are those in which the metal cation is an alkali metal cation such as sodium, potassium, lithium or the like; alkaline earth metal cation such as calcium, magnesium, barium, strontium or the like; or heavy metal cation such as zinc, manganese, cupric, cuprous, ferric, ferrous, titanium, aluminum or the like. The ammonium salts include those in which the ammonium cation has the formula $NR^5R^6R^7R^8$ wherein each of $R^5$, $R^6$, $R^7$ and $R^8$ are independently hydrogen, hydroxy, $(C_1-C_4)$alkoxy, $(C_1-C_{20})$alkyl, $(C_{3-8})$alkenyl, $(C_3-C_8)$alkynyl, $(C_2-C_8)$hydroxyalkyl, $(C_2-C_8)$alkoxyalkyl, $(C_2-C_6)$aminoalkyl, $(C_2-C_6)$haloalkyl, amino, $(C_1-C_4)$-alkyl- or $(C_1-C_4)$dialkylamino, substituted or unsubstituted phenyl, substituted or unsubstituted phenylalkyl, having up to four carbon atoms in the alkyl moiety, or any two of $R^5$, $R^6$, $R^7$ or $R^8$ can be taken together to form with the nitrogen atom a 5- or 6-membered heterocyclic ring, optionally having up to one additional hetero atom (e.g., oxygen, nitrogen, or sulfur) in the ring, and preferably saturated, such as piperidino, morpholino, pyrrolidino, piperazino or the like, or any three of $R^5$, $R^6$, $R^7$ or $R^8$ can be taken together to form with the nitrogen atom a 5- or 6-membered aromatic heterocyclic ring, such as piperazole or pyridine. When $R^5$, $R^6$, $R^7$ or $R^8$ substituent in the ammonium group is a substituted phenyl or substituted phenylalkyl, the substituents on the phenyl and phenalkyl will generally be selected from halo, $(C_1-C_8)$alkyl, $(C_1-C_4)$alkoxy, hydroxy, nitro, trifluoromethyl, cyano, amino, $(C_1-C_4)$alkylthio and the like. Such substituted phenyl groups preferably have up to two such substituents. Representative ammonium cations include ammonium, dimethylammonium, 2-ethylhexylammonium, bis(2-hydroxyethyl)ammonium, tris(2-hydroxyethyl)ammonium, dicyclohexylammonium, t-octylammonium, 2-hydroxyethylammonium, morpholinium, piperidinium, 2-phenethylammonium, 2-methylbenzylammonium, n-hexylammonium, triethylammonium, trimethylammonium, tri(n-butyl)ammonium, methoxyethylammonium, diisopropylammonium, pyridinium, dialkylammonium, pyrazolium, propargylammonium, dimethylhydrazinium, octadecylammonium, 4-dichlorophenylammonium, 4-nitrobenzylammonium, benzyltrimethylammonium, 2-hydroxyethyldimethyloctadecylammonium, 2-hydroxyethyldiethyloctylammonium, decyltrimethylammonium, hexyltriethylammonium, 4-methylbenzyltrimethylammonium, and the like. Among the acid addition salts are those in which the anion is an agronomically acceptable anion such as hydrochloride, hydrobromide, sulfate, nitrate, perchlorate, acetate, oxalate and the like.

The compounds of this invention or their precursors can be prepared according to the following processes.

Process A:

Step 1

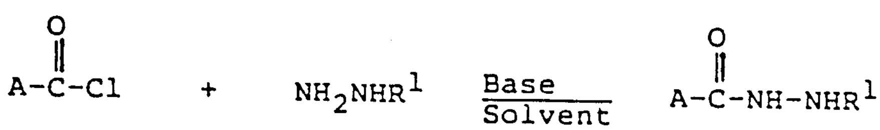


II III IV

Step 2

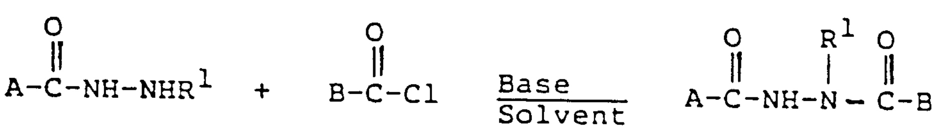


IV V I

where $R^1$, A and B are as defined above for Formula I and X and X' are oxygen.

In process A, a compound of Formula II is reacted with a monosubstituted hydrazine of Formula III or a corresponding acid addition salt such as the hydrochloride salt or the like, in the presence of a base in an inert or substantially inert solvent or mixture of solvents to afford an intermediate product of Formula IV which can be isolated or further reacted with a compound of Formula V in the presence of a base in an inert or substantially inert solvent or mixture of solvents to afford the desired product of Formula I.

Examples of the compounds of Formula II which can be used in the above processes include benzoyl chloride, 4-chlorobenzoyl chloride, 4-methylbenzoyl chloride, 3,5-dichlorobenzoyl chloride, 2-bromobenzoyl chloride, 3-cyanobenzoyl chloride adamantave carbonyl chloride and the like.

Examples of the compounds of Formula V which can be used in the above processes include benzoyl chloride, 4-chlorobenzoyl chloride, 4-methylbenzoyl chloride, 3,5-dichlorobenzoyl chloride, 2-bromobenzoyl chloride, 3-cyanobenzoyl chloride, and the like.

The compounds of Formula II and/or Formula V are generally commercially available or can be prepared by known procedures.

Examples of the compounds of Formula III which can be used in the above processes include isopropylhydrazine, t-butylhydrazine, neopentylhydrazine, alpha-methylneopentylhydrazine, isobutylhydrazine, isopentylhydrazine, isooctylhydrazine, and the like. The compounds of Formula III are generally commercially available or can be prepared by known procedures.

Suitable solvents for use in the above processes include water; alcohols such as methanol, ethanol, isopropanol and the like; hydrocarbons such as toluene, xylene, hexane, heptane and the like; glyme; tetrahydrofuran; acetonitrile; pyridine; or haloalkanes such as methylene chloride or mixtures of these solvents.

Preferred solvents are water, toluene, methylene chloride or a mixture of these solvents.

Examples of bases for use in the above processes include tertiary amines such as triethylamine; pyridine; potassium carbonate; sodium carbonate; sodium bicarbonate; sodium hydroxide; or potassium hydroxide. Preferred bases are sodium hydroxide, potassium hydroxide or triethylamine.

The above processes can be carried out at temperatures between about -20 °C and about 100 °C. Preferably, these reactions are carried out between about -5 °C and about 50 °C.

Preparation of the compounds of the present invention is generally carried out at about atmospheric pressure although higher or lower pressures can be used if desired.

Substantially equimolar amounts of reactants are preferably used although higher or lower amounts can be used if desired.

Generally, about one equivalent of base is used per equivalent of starting material of Formula II, V, XI and/or XIII. Where the acid addition salt of the mono-substituted hydrazine of Formula III is used, one additional equivalent of base is used. For example, when substituents A and B are different and an acid addition salt of the monosubstituted hydrazines of Formula III is used, about two equivalents of base are used in Step 1 and about one equivalent of base is used in Step 2.

Modifications to the above process may be necessary to accommodate reactive functionalities of particular A and/or B substituents. Such modifications would be apparent and known to those skilled in the art.

The agronomically acceptable salts embraced by Formula I of the invention can be prepared by reacting a metal hydroxide, a metal hydride or an amine or ammonium salt, such as a halide, hydroxide or alkoxide with a compound of Formula I having one or more hydroxy or carboxy groups or reacting a quaternary ammonium salt, such as chloride, bromide, nitrate or the like with a metal salt of a compound of Formula I in a suitable solvent. When metal hydroxides are used as reagents, useful solvents include water; ethers such as glyme and the like; dioxane; tetrahydrofuran; alcohols such as methanol, ethanol, isopropanol and the like. When metal hydrides are used as reagents, useful solvents include nonhydroxylic solvents, for example, ethers such as dioxane, glyme, diethylether and the like; tetrahydrofuran; hydrocarbons such as toluene, xylene, hexane, pentane, heptane, octane and the like; dimethylformamide, and the like. When amines are used as reagents, useful solvents include alcohols, such as methanol or ethanol; hydrocarbons, such as toluene, xylene, hexane and the like; tetrahydrofuran; glyme; dioxane; or water. When ammonium salts are used as reagents, useful solvents include water; alcohols, such as methanol or ethanol; glyme; tetrahydrofuran; or the like. When the ammonium salt is other than a hydroxide or alkoxide, an additional base, such as potassium or sodium hydroxide, hydride, or alkoxide is generally used. The particular choice of solvent will depend on the relative solubilities of the starting materials and the resultant salts, and slurries rather than solutions of certain reagents may be used to obtain the salts. Generally, equivalent amounts of the starting reagents are used and the salt-forming reaction is carried out at about 0 °C to about 100 °C, preferably at about room temperature.

The acid addition salts of the present invention can be prepared by reacting hydrochloric, hydrobromic, sulfuric, nitric, phosphoric, acetic, propionic, benzoic or other suitable acid with a compound of Formula I having a basic functional group in a suitable solvent. Useful solvents include water, alcohols, ethers, esters, ketones, haloalkanes and the like. The particular choice of solvent will depend on the relative solubilities of the starting materials and the resulting salts and slurries ratner than solutions of certain reagents may be used to obtain the salts. Generally, equivalent molar amounts of starting materials are used and the salt-forming reaction is carried out at from about -10 °C to about 100 °C, preferably at about room temperature.

The following examples will further illustrate this invention but are not intended to limit it in any way. In Table I, some N′-substituted-N-alkylcarbonyl-N′-acylhydrazines and N′-substituted-N-acyl-N′-alkylcarbonylhydrazines of the present invention that have been made are listed. Structures were confirmed by NMR and in some cases by IR and/or elemental analysis. Specific illustrative preparation of the compounds of Examples 1 and 3 are described after Table I.

TABLE I

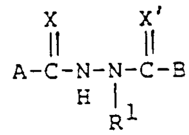

| Ex. No. | X | X' | $R^1$ | A | B | m.p. °C |
|---|---|---|---|---|---|---|
| 1 | O | O | $-C(CH_3)_3$ | $-C_6H_5$ | 1-adamantyl | 209-212 |
| 2 | O | O | $-C(CH_3)_3$ | 1-adamantyl | $-C_6H_4(CH_3)-3$ | 185 |
| 3 | O | O | $-C(CH_3)_3$ | 1-adamantyl | $-C_6H_5$ | 228 |
| 4 | O | O | $-C(CH_3)_3$ | 1-adamantyl | $-C_6H_3(CH_3)_2-3,5$ | 221 |
| 5 | O | O | $-C(CH_3)_3$ | 1-adamantyl | $-C_6H_4Br-2$ | 186-187 |
| 6 | O | O | $-C(CH_3)_3$ | 1-adamantyl | $-C_6H_3Cl_2-3,5$ | 215 |
| 7 | O | O | $-C(CH_3)_3$ | 1-adamantyl | $-C_6H_3Cl_2-2,4$ | 174 |
| 8 | O | O | $-C(CH_3)_3$ | 1-adamantyl | $-C_6H_4NO_2-2$ | 195-197 |
| 9 | O | O | $-C(CH_3)_3$ | 1-adamantyl | $-C_6H_3Cl-2-F-4$ | 179-181 |

Example 1 - Prepration of N′-t-Butyl-N-Benzoyl-N′-(1-adamantane)-carbonylhydrazine

To a stirred suspension of t-butylhydrazine hydrochloride (1 g, 0.008 M) in toluene (30 ml) at room temperature was added dropwise a 50% aqueous solution of sodium hydroxide (0.64 g, 0.008M). After 15 minutes, the reaction mixture was cooled to 5 °C and a solution of benzoyl chloride (1.12 g, 0.008) in toluene (5 ml) and a solution of aqueous 50% sodium hydroxide (0.64 g, 0.008M) were added dropwise simultaneously from separate addition funnels while maintaining the temperature at or below 10 °C. Following the addition, the reaction mixture was warmed to room temperature and stirred for 1 hour. The reaction was diluted with hexane and the solid N′-t-butyl-N-benzoylhydrazine was isolated by filtration.

To a stirred, cooled (0 °C) slurry of 0.5 g of N′-t-butyl-N-benzoylhydrazine in 30 ml of toluene was added simultaneously but separately a solution of 0.6 g of 1-adamantanecarbonyl chloride in 20 ml of toluene and 0.2 g of an aqueous 50% sodium hydroxide solution. After the additions the mixture was allowed to warm to room temperature and stir for 12 hours. A white solid precipitated from the solution which was collected by filtration to give N′-t-butyl-N-benzoyl-N′-(1-adamantane)carbonylhydrazine (m.p. 209-212 °C).

Example 3 - Preparation of N′-butyl-N′-Benzoyl-N-(1-adamantane)- carbonylhydrazine

To a stirred suspension of t-butylhydrazine hydrochloride (1 g, 0.008M) in toluene (30 ml) at room temperature was added dropwise a 50% aqueous solution of sodium hydroxide (0.64 g, 0.008M). After 15 minutes, the reaction mixture was cooled to 5 °C and a solution of 1-adamantanecarbonyl chloride (1.6 g, 0.008) in toluene (5 ml) and a solution of aqueous 50% sodium hydroxide (0.64 g, 0.008M) were added dropwise simultaneously from separate addition funnels while maintaining the temperature at or below

10 ° C. Following the addition, the reaction mixture was warmed to room temperature and stirred for 1 hour. The reaction was diluted with hexane and the solid N′-t-butyl-N-adamantanecarbonylhydrazine was isolated by filtration.

A 25 ml erlenmeyer was charged with 2.4 g. of water, 0.65 g. 50% sodium hydroxide, 10 ml of methylene chloride and 1.0 g 1-adamantyl-N′-t-butylhydrazide. To this rapidly stirring solution 0.64 g of benzoyl chloride in 5 ml methylenechloride was added dropwise. The solution was stirred for 2 hours, then water and methylene chloride were added. The separated organic phase was washed with sat. NaCl, dried $MgSO_4$, and concentrated in vacuum. The concentrate was taken up in boiling ether, cooled and filtered to yield 1.32 g m.p. 228 ° C.

By following substantially the procedure as exemplified above by the preparation of the compounds of Examples 1, 3, the compounds of Table 1 were prepared.

As previously noted, the compounds of the present invention exhibit excellent pesticidal activity and are selective against insects of the orders Lepidoptera and Coleoptera.

In general, for the control of insects in agriculture horticulture and forestry, a dosage corresponding to from about 10 g to about 10 kg of the active substance per hectare may be used and from about 100 g to about 5 kg per hectare of the active substance is preferred. The exact amount of dosage for a given situation can be routinely determined and depends on a variety of factors, for example, the substance used, the kind of pest, the formulation used, the state of the crop infested with the pest and the prevailing weather conditions. The term "insecticidal" as employed in the specification and claims of this application is to be construed as any means which adversely affects the existence or growth of the target insects. Such means can comprise a complete killing action, eradication, arresting in growth, inhibition, reducing in number or any combination thereof. The term "control" as employed in the specification and claims of this application is to be construed as meaning "insecticidal" or protecting plants from insect damage. By "insecticidally effective amount" is meant that dosage of active substance sufficient to exert insect "control."

The compounds of the present invention, for practical applications, can be utilized in the form of compositions or formulations. Examples of the preparation of compositions and formulations can be found in the American Chemical Society publication "Pesticidal Formulation Research," (1969), Advances in Chemistry Series No. 86, written by Wade Van Valkenburg; and the Marcel Dekker, Inc. publication "Pesticide Formulations," (1973), edited by Wade Van Valkenburg. In these compositions and formulations, the active substance is mixed with conventional inert agronomically acceptable (i.e., plant compatible and/or pesticidally inert) diluents or extenders such as solid carrier material or liquid carrier material, of the type usable in conventional compositions or formulations. By agronomically acceptable carrier is meant any substance which can be used to dissolve, disperse or diffuse the active ingredient in the composition without impairing the active ingredient's effectiveness and which by itself has no significant detrimental effect on the soil, equipment, desirable plants or agronomic environment. If desired, adjuvants such as surfactants, stabilizers, antifoam agents and antidrift agents may also be added.

Examples of compositions and formulations according to the invention are aqueous solutions and dispersions, oily solutions and oil dispersions, pastes, dusting powders, wettable powders, emulsifiable concentrates, flowables, granules, baits, invert emulsions, aerosol compositions and fumigating candles.

Wettable powders, pastes, flowables and emulsifiable concentrates are concentrated preparations which are diluted with water before or during use.

Baits are preparations generally comprising a food or other substance attractive to the target pest, that includes at least one lethal or non-lethal toxicant. Lethal toxicants kill the pest upon ingesting the bait while non-lethal toxicants change the behavior, feeding habits and physiology of the pest for the purpose of control.

The invert emulsions are mainly used for aerial application, where large areas are treated with a comparatively small amount of preparation. The invert emulsion may be prepared in the spraying apparatus shortly before, or even during, the spraying operation by emulsifying water in an oil solution or an oil dispersion of the active substance.

Compositions and formulations are prepared in a known manner, for instance by extending the active compounds with. conventional dispersible liquid diluent carriers and/or dispersible solid carriers optionally with the use of carrier vehicle assistants, e.g., conventional surface-active agents, including emulsifying agents and/or dispersing agents, whereby, for example, in the case where water is used as diluent, organic solvents may be added as auxiliary solvents. The following may be chiefly considered for use as conventional carrier vehicles for this purpose: aerosol propellants which are gaseous at normal temperatures and pressures, such as halogenated hydrocarbons, e.g., dichlorodifluoromethane and trifluorochloromethane, as well as butane, propane, nitrogen and carbon dioxide; inert dispersible liquid diluent carriers, including inert organic solvents, such as aromatic hydrocarbons (e.g., benzene, toluene, xylene,

alkyl naphthalenes, etc.), halogenated, especially chlorinated, aromatic hydrocarbons (e.g., chlorobenzenes, etc.), cycloalkanes (e.g., cyclohexane, etc.), paraffins (e.g., petroleum or mineral oil fractions), chlorinated aliphatic hydrocarbons (e.g., methylene chloride, chloroethylenes, etc.), vegetable oils (e.g., soybean oil, cottonseed oil, corn oil, etc.), alcohols (e.g., methanol, ethanol, propanol, butanol, glycol, etc.) as well as ethers and esters thereof (e.g., glycol monomethyl ether, etc.), amines (e.g., ethanolamine, etc.), amides (e.g., dimethyl formamide, etc.), sulfoxides (e.g., dimethyl sulfoxide, etc.), acetonitrile, ketones (e.g., acetone, methyl ethyl ketone, methyl isobutyl ketone, cyclohexanone, isophorone, etc.), and/or water; solid carriers including ground natural minerals, such as kaolins, clays, talc, chalk, quartz, attapulgite, montmorillonite or diatomaceous earth, and ground synthetic minerals, such as highly-dispersed silicic acid, alumina and silicates; solid carriers for granules include crushed and fractionated natural rocks such as calcite, marble, pumice, sepiolite and dolomite, as well as synthetic granules of inorganic and organic meals, and granules of organic material such as sawdust, coconut shells, corn cobs and tobacco stalks. The following may be chiefly considered for use as conventional carrier vehicle assistants: emulsifying agents, such as cationic and/or nonionic and/or anionic emulsifying agents (e.g., polyethylene oxide esters of fatty acids, polyethylene oxide ethers of fatty alcohols, alkyl sulfates, alkyl sulfonates, aryl sulfonates, albumin hydrolysates, etc., and especially alkyl arylpolyglycol ethers, magnesium stearate, sodium oleate, etc.); and/or dispersing agents, such as lignin, sulfite waste liquors, methyl cellulose, etc.

Adhesives such as carboxymethylcellulose and natural and synthetic polymers in the form of powders, granules or latices, such as gum arabic, polyvinyl alcohol and polyvinyl acetate, can be used in the formulations.

If desired, it is possible to use colorants in compositions and formulations containing compounds of the present invention such as inorganic pigments, for example, iron oxide, titanium oxide and Prussian Blue, and organic dyestuffs, such as alizarin dyestuffs, azo dyestuffs and metal phthalocyanine dyestuffs, and trace nutrients such as salts of iron, manganese, boron, copper, cobalt, molybdenum and zinc.

The active compounds of the present invention may be employed alone or in the form of mixtures with one another and/or with such solid and/or liquid dispersible carrier vehicles and/or with other known compatible active agents, especially plant protection agents, such as other insecticides, arthropodicides, nematicides, fungicides, bactericides, rodenticides, herbicides, fertilizers, growth-regulating agents, synergists, etc., if desired, or in the form of particular dosage preparations for specific application made therefrom, such as solutions, emulsions, suspensions, powders, pastes and granules which are thus ready for use.

As concerns commercially marketed preparations, these generally contemplate carrier composition mixtures in which the active compound is present in an amount substantially between about 0.1% and 99% by weight, and preferably between about 1% and 75% by weight, of the mixture. Carrier composition mixtures suitable for direct application or field application generally contemplate those in which the active compound is used in an amount substantially between about 0.0001% and 5%, preferably between about 0.001% and 3%, by weight of the mixture. Thus the present invention contemplates overall formulations and compositions which comprise mixtures of a conventional dispersible carrier such as (1) a dispersible inert, finely divided carrier solid, and/or (2) a dispersible carrier liquid such as an inert organic solvent and/or water, preferably including a surface-active effective amount of a carrier vehicle assistant (e.g., a surface-active agent, such as an emulsifying agent and/or a dispersing agent), and an amount of the active compound generally between about 0.0001% and about 99% by weight of the composition, preferably between about 0.001% and about 90% by weight of the composition, and more preferably between about 0.01% and about 75% by weight of the composition which is effective for the purpose in question.

The active compounds can be applied as sprays by methods commonly employed, such as conventional high-gallonage hydraulic sprays, low gallonage sprays, ultra-low-volume sprays, airblast spray, aerial sprays and dusts. If low volume applications are desired, a solution of the compound is usually used. In ultra-low-volume applications, a liquid composition containing the active compound is usually applied as a spray (e.g., mist) by means of atomizing equipment in finely divided form (average particle size of from about 50 to about 100 microns or less) using airplane crop spraying techniques. Typically only a few liters per hectare are needed and often amounts up to about 15 to 1000 g/hectare, preferably about 40 to 600 g/hectare are sufficient. With ultra-low-volume, it is possible to use highly concentrated liquid compositions with said liquid carrier vehicles containing from about 20 to about 95% by weight of the active compound.

Furthermore, the present invention contemplates methods of selectively killing, combatting or controlling pests, which comprises contacting insects with a correspondingly combative or toxic amount (i.e., an insecticidally effective amount) of at least one active compound of the invention alone or together with a carrier vehicle (composition or formulation) as noted above. The term "contacting" as employed in the specification and claims of this application is to be construed as applying to at least one of (a) such insects

and (b) the corresponding habitat thereof i.e., the locus to be protected, for example, to a growing crop or an area where a crop is to be grown) the active compound of this invention alone or as a constituent of a composition or formulation. The instant formulations or compositions are applied in the usual manner, for instance by spraying, atomizing, vaporizing, scattering, dusting, watering, squirting, sprinkling, pouring, fumigating, dry dressing, moist dressing, wet dressing, slurry dressing, encrusting and the like.

The terms "control" and "combat" as employed in the specification and Claims of this application are to be construed as including "insecticidal" and the protection of plants from insect damage. By "insecticidally effective amount" or pesticidally effective amount" is meant that dosage of active substance sufficient to exert insect "control" or to "combat" insects.

It will be realized, of course, that the concentration of the particular active compound utilized in admixture with the carrier vehicle will depend upon such factors as the type of equipment employed, method of application, area to be treated, types of pests to be controlled and degree of infestation. Therefore, in special cases it is possible to go above or below the aforementioned concentration ranges.

Granular preparations are produced, for example, by taking up the active substance in a solvent and by using the resulting solution, as the case may be in the presence of a binder, to impregnate a granular carrier material, such as porous granules (for example, pumice and attaclay), or chopped tobacco stems or the like.

A granular preparation (frequently termed a "pellet") may alternatively be produced by compressing the active substance together with powdered minerals in the presence of lubricants and binders and by disintegrating and straining the composite to the desired grain size.

Dusts are obtainable by intimately mixing the active substance with an inert solid carrier material in a concentration of from about 1 to about 50% by weight. Examples of suitable solid carrier materials are talc, kaolin, pipe clay, diatomaceous earth, dolomite, gypsum, chalk, bentonite, attapulgite and colloidal $SiO_2$ or mixtures of these and similar substances. Alternatively organic carrier materials such as, for example, ground walnut shells may be used.

Wettable powders and flowables are produced by mixing from about 10 to about 99 parts by weight of a solid inert carrier such, for example, as the aforementioned carrier materials with from about 1 to about 80 parts by weight of the active substance optionally dissolved in a volatile solvent such as acetone, from about 1 to about 5 parts by weight of a dispersing agent such, for example, as the lignosulfonates or alkylnaphthalene sulfonates known for this purpose and preferably also from about 0.5 to about 5 parts by weight of a wetting agent, such as fatty alcohol sulfates, or alkylarylsulfonates of fatty acid condensation products. In the case of flowables, a liquid inert carrier such as water is also included.

To produce emulsifiable concentrates the active compound is dissolved or finely divided in a suitable solvent which preferably is poorly miscible with water, an emulsifier being added to the resulting solution. Examples of suitable solvents are xylene, toluene, high-boiling aromatic petroleum distillates, for example, solvent naphtha, distilled tar oil and mixtures of these liquids. Examples of suitable emulsifiers are alkylphenoxypolyglycol ethers, polyoxyethylene sorbitan esters of fatty acids or polyoxyethylene sorbitol esters of fatty acids. The concentration of the active compound in these emulsifiable concentrates is not restricted within narrow limits and may vary between about 2% and about 50% by weight depending upon toxicant solubility. A suitable liquid, highly concentrated primary composition other than an emulsifiable concentrate is a solution of the active substance in a liquid which is readily miscible with water, for example, acetone, to which solution a dispersant and, as the case may be, a wetting agent are added. When such a primary composition is diluted with water shortly before or during the spraying operation an aqueous dispersion of the active substance is obtained.

An aerosol preparation according to the invention is obtained in the usual manner by incorporating the active substance or a solution thereof in a suitable solvent in a volatile liquid suitable for use as a propellant such, for example, as a mixture of chlorine and fluorine derivatives of methane and ethane.

Fumigating candles or fumigating powders, i.e., preparations which when burning are capable of emitting a pesticidal smoke., are obtained by taking up the active substance in a combustible mixture which may, for example, comprise a sugar or a wood, preferably in the ground form, as a fuel, a substance to sustain combustion such, for example, as ammonium nitrate or potassium chlorate, and furthermore a substance for retarding combustion, for example, kaolin, bentonite and/or colloidal silicic acid.

A bait preparation comprises a food or other substance attractive to pests, a carrier, the toxicant and may optionally include other substances commonly used in preparations of this kind, such as, a preservative to inhibit bacterial and fungal growth, a waterproofing agent to prevent disintegration under wet conditions and dyes or colorants as described above.

In addition to the aforementioned ingredients, the preparations according to the invention may also contain other substances commonly used in preparations of this kind.

For example, a lubricant, such as calcium stearate or magnesium stearate, may be added to a wettable powder or to a mixture to be granulated. Furthermore, there may, for example, be added "adhesives" such as polyvinylalcohol cellulose derivatives or other colloidal materials, such as casein, to improve the adherence of this pesticide to the surface to be protected.

In another aspect of the present invention, there is provided a vendible article which comprises:

1) a container,

(2) an insecticidally active compound as hereinbefore defined, optionally in a composition as hereinbefore defined. Preferably, the vendible article additionally comprises

(3) instructing information comprising written and/or illustrative information for applying the active compound to either growing plants, or seeds, or an area where or a medium in which plants are to be grown or are growing. The active compound may be in a composition which also comprises agronomically acceptable diluent or carrier. The composition may contain another different compound which is also effective at combating insect infestation in plants. The container is preferably a box, pail, drum, bottle or canister. Preferably, the instructing information is on an outside surface of the container, for example on the outside surface of the wall, lid or base of a box, pail or drum. The instructing information may be applied directly to the outside surface of the container or said information may be on a label adhered to an outside surface of the container. Alternatively, the instructing information may be on an inside surface of the container; for example the information may be on the inner surface of the lid of a box, pail or drum; or on an inner surface of a wall of a cardboard box (in which case the active compound will probably have to be removed from the box, and the box possibly dismantled, for the instructions to be read); or the walls of the container may be transparent and the instructions read from the outside. In another embodiment, the instructing information is on a sheet of paper, or other suitable substrate, which may be either contained within the container; or detachably, but not permanently, attached to an outer surface of the container e.g. the information may be on a sheet of paper held within an envelope which is adhered to the outside of the container, or simply held to the container by a rubber band.

The present invention also concerns a method of mixing an insecticidally active compound as hereinbefore defined with an agronomically acceptable diluent or carrier. The composition produced by this method of mixing preferably is a composition as hereinbefore defined.

Representative preparation of compositions and formulations including the compounds of the present invention are set forth below as Examples A through I by way of illustration but not limitation.

<u>Example A</u>

| Granular | |
|---|---|
| Ingredient | %/wt. |
| Toxicant and toxicant impurities | 0.25 |
| Triton® X-305 (binder) (Octylphenyl-30-ethylene oxide ethanol) | 0.25 |
| Agsorb® 24/48 (diluent) (Montmorillonite clay) | 99.50 |

Preparation: The toxicant and Triton® X-305 are dissolved into methylene chloride and the mixture is added to the Agsorb® with continuous mixing. The methylene chloride is then allowed to evaporate.

<u>Example B</u>

| Dust | |
|---|---|
| Ingredient | %/wt. |
| Toxicant and toxicant impurities | 1.0 |
| Talc | 99.0 |

Preparation: Toxicant is dissolved in excess acetone and the mixture is impregnated onto the talc. The acetone is then permitted to evaporate.

Example C

| Wettable Powder | |
|---|---|
| Ingredient | %/wt. |
| Toxicant and toxicant impurities | 31.3 |
| Duponal® WA Dry (wetter) (Sodium lauryl sulfate) | 2.0 |
| Reax® 45A (dispersant) (Sodium lignin sulfonate) | 5.0 |
| Barden clay (diluent) | 31.7 |
| HiSil® 233 (diluent) (Sodium silica) | 30.0 |

Preparation: The toxicant, optionally dissolved in a volatile solvent, is absorbed onto the Barden clay and HiSil® carriers. The Duponal® and Reax® are then added and the entire dry mixture blended until homogeneous. The composition is then micronized to a fine particle size.

Example D

| Emulsifiable Concentrate | |
|---|---|
| Ingredient | %/wt. |
| Toxicant and toxicant impurities | 15.0 |
| Sponto® 232T (emulsifier) (Anionic and nonionic blend of the following surfactants: calcium dodecyl benzene sulfonate; and ethoxylated alkylphenol) | 6.0 |
| Sponto® 234T (emulsifier) (Anionic and nonionic blend of the following surfactants: calcium dodecyl benzene sulfonate; and ethoxylated alkylphenol) | 4.0 |
| Cyclohexanone (solvent) | 22.5 |
| Tenneco® 500-100 (solvent) (Aromatic solvent mixture principally comprising xylene, cumene and ethyl benzene having a boiling point range of 290-345 °F) | 52.5 |

Preparation: All ingredients are mixed together with continuous agitation until a homogeneous clear solution is obtained.

Example E

| Aerosol | |
|---|---|
| Ingredient | %/wt. |
| Toxicant and toxicant impurities | 0.5 |
| Freon 12 | 99.5 |

Preparation: The components are mixed and packaged under pressure in a suitable container equipped with a release spray valve.

Example F

| Fumigating Candle or Fumigating Powder | |
|---|---|
| Ingredient | %/wt. |
| Toxicant and toxicant impurities | 1.0 |
| Wood dust | 96.0 |
| Starch | 3.0 |

Preparation: Toxicant, wood dust, and starch are blended together and then molded into a candle using a small amount of water to activate the starch.

Example G

Bait

Method A

| Ingredient | %/wt. |
|---|---|
| Toxicant and toxicant impurities | 1.00 |
| Wheat Bran (carrier and attractant) | 89.95 |
| Corn Syrup (attractant) | 7.00 |
| Corn Oil (attractant) | 2.00 |
| Kathon® 4200 (preservative) (3-isothiazolone) | 0.05 |

Preparation: The corn oil and corn syrup are added to the wheat bran with adequate mixing. The toxicant and Kathon® are premixed with excess acetone and this solution is added to the wheat bran base with continued mixing. The acetone is then permitted to evaporate.

Method B

| Ingredient | %/wt. |
|---|---|
| Toxicant and toxicant impurities | 0.06 |
| Granulated Sugar (carrier and attractant) | 99.94 |

Example H

Pellet

Same as Example G, Method A, with this addition: the bait composition is formed into 1/4" diameter by 3/8" long pellets using a suitable die and press apparatus.

Example I

| Flowable | |
|---|---|
| Ingredient | %/wt. |
| Toxicant and toxicant impurities | 31.3 |
| Duponal® WA Dry (wetter) (Sodium lauryl sulfate) | 2.0 |
| Reax® 45A (dispersant) (Sodium lignin sulfonate) | 5.0 |
| HiSil® 233 (diluent) (Sodium silica) | 30.0 |
| Kelzan® (thickener) (Xanthan gum) | 0.5 |
| Water | 31.2 |

Preparation: The toxicant is absorbed into the HiSil® carrier. The Duponal® and Reax® are then added and the entire dry mixture blended until homogeneous. The composition is then micronized to a fine particle size. The resulting powder is suspended in water and the Kelzan® added.

Compositions and formulations according to the present invention may also include known pesticidal compounds. This expands the spectrum of activity of the preparations and may give rise to synergism.

The following known insecticidal, fungicidal and acaricidal compounds are suitable for use in such a combined preparation.

Insecticides such as:

Chlorinated hydrocarbons, for example, 2,2-bis(p-chlorophenyl)-1,1,1-trichloroethane and hexachloroepoxyoctahydrodimethanonaphthalene;

Carbamates, for example, 2-sec-butyl phenyl methyl carbamate; Pyrethroids, for example, 2-(4-ethoxy phenyl)-2-methyl propyl 3-phenoxybenzyl ether;

Alkylenebisdithiocarbamates, for example, zinc ethylenebisthiocarbamate and manganese ethylenebisdithiocarbamate;

2,4-dinitro-6-(2'octyl-phenylcrotonate), 1-bis(dimethylamino)phosphoryl-3-phenyl-5-amino-1,2,4-triazole, 6-methylquinoxaline-2,3-dithiocarbonate, 1,4-dithioanthraquinone-2,3-dicarbonitrite, N-trichloromethylthiophthalimide, N-trichloromethylthiotetrahydrophthalimide, N-(1,1,2,2-tetrachloroethylthio)-tetrahydrophthalimide, N-dichlorofluoromethylthio-N-phenyl-N'-dimethylsulfonyldiamide and tetrachloroisophthalonitrile;

Insect growth regulators, for example, 2-tert-butylimino-3-isopropyl-5-phenyl-3,4,5,6-tetrahydro-2H-1,3,5-thiadiazin-4-one; and

Fungicides, for example, α,α,α-trifluoro-3'-isopropoxy-O-toluanilide, diisopropyl-1,3-di thiolan-2-ylidenemalonate, 5-methyl-1,2,4-triazolo-(3,4-b)-benzothiazole, 3-allyloxy-1,2-benzoisothiazole 1,1-dioxide.

Biological Activity

It has been found by biological evaluation that compounds according to the present invention have pesticidal activity and are capable of controlling larvae and adult forms of pests, especially insects from the orders Lepidoptera and Coleoptera. One skilled in the art will know how to determine the activity of a given compound against a given insect and the dosage required to obtain general or selective insecticidal effects.

As previously noted, the compounds of the present invention are particularly suitable for controlling plant-destructive insects in crops of cultivated plants, such as, but not limited to, cotton, vegetables, corn and other cereals, and the like; forestry, such as, but not limited to, birch, spruce, pine, fir, and the like; and ornamental plants, flowers and trees. Compounds of the present invention are also Carbamates, for example, N-methyl-1-naphthylcarbamate;

Dinitrophenols, for example, 2-methyl-4,6-dinitrophenol and 2-(2-butyl)-4,6-dinitrophenyl-3,3-dimethylacrylate;

Organic phosphorus compounds, such as dimethyl-2-methoxy-3-carbonyl-1-methylvinyl phosphate, 0,0-diethyl-0-p-nitrophenylphosphorothioate; N-monomethylamide of 0,0-dimethyldithiophosphorylacetic acid;

Diphenylsulfides, for example, p-chlorobenzyl or p-chlorophenyl sulfide and 2,4,4',5-tetrachlorodiphenylsulfide;

Diphenylsulfonates, for example, p-chlorophenylbenzenesulfonate;

Methylcarbinols, for example, 4,4-dichloro-1-trichloromethylbenzhydrol;

Quinoxaline compounds, such as methylquinoxaline dithiocarbonate;

Amidines such as N'-(4-chloro-2-methylphenyl) N,N-dimethylformamidine;

Pyrethroids such as Allethrin;
Biologicals such as Bacillus thuringiensis preparations;
Organic tin compounds such as tricyclohexyltin hydroxide;
Synergists such as piperonyl butoxide.

Fungicides such as:
Organic mercury compounds, for example, phenylmercuryacetate and methylmercurycyanoguanide;
Organic tin compounds, for example, triphenyltin hydroxide and triphenyltin acetate;
particularly suitable for controlling insects destructive to stored commodities such as seeds and the like; fruit crops, such as, but not limited to, fruit and/or citrus trees, raspberry bushes, and the like; and turf, such as, but not limited to, lawns, sod and the like.

In evaluating the pesticidal activity of the compounds of this invention, the following test procedures were employed.

A test solution containing 600 parts per million (ppm) was made by dissolving the test compound in a solvent (acetone:methanol, 1:1), adding water to give an acetone:methanol:water system of 5:5:90 and then a surfactant. A 1:1 mixture of an alkylarylpolyetheralcohol (sold under the trademark Triton® X-155) and a modified phthalic glycerol alkyl resin (sold under the trademark Triton® B-1956) was utilized at the equivalent of 1 ounce per 100 gal. of test solution as a surfactant.

Initial evaluations were made on one or more of the following pests:

| Code Symbol | Common Name | Latin Name |
|---|---|---|
| SAW | Southern Armyworm | Spodoptera eridania |
| MBB | Mexican Bean Beetle | Epilachna varivestis |

For the foliar bean beetle and armyworm tests, individual bean (Phaseolus limensis var. Woods' Prolific) leaves are placed on moistened pieces of filter paper in Petri dishes. The leaves are then sprayed with the test solution using a rotating turntable and allowed to dry. The dishes are infested with 10 third instar larvae of Southern armyworm or Mexican bean beetle. The dishes are then covered.

The percent mortality for the bean beetle and armyworm evaluations are determined 96 hours after treatment. Evaluations are based on a scale of 0-100% in which 0 equals no activity and 100 equals total kill.

The rotating turntable consists of a fixed, continuously operating spray nozzle under which targets are rotated at a fixed speed and distance. When the target is a Petri dish (such as for the armyworm), the distance from the nozzle is 15 inches. The nozzle is located 8 inches from the rotating shaft. The targets on individual platforms revolve around the shaft at 1 revolution per 20 seconds but only a brief portion of this time occurs in the spray path. Targets pass only once under the nozzle and then are removed to drying hoods.

The nozzle used is a 1/4 JCO Spraying Systems (Wheaton, Illinois) air atomizing nozzle equipped with a No. 2850 fluid cap and No. 70 air cap. At the 10 psig air pressure used and with liquid siphon feed 0.5 GPH (gallons per hour) are delivered in a round spray pattern with a 21° spray angle. Targets are misted with spray droplets to the point that the droplets coalesce to form a uniform thin film insufficient to drown test organisms.

All treatments are maintained at 75-80°F under continuous fluorescent light in a well-ventilated room.

For soil treatment (systemic) trials, a portion of the 600 ppm test solution is diluted to 150 ppm. Ten (10) ml of the 150 ppm test solution is pipetted into soil (approximately 200 g of standard greenhouse soil) in a 3-inch pot containing a lima bean seedling. This results in a soil concentration of approximately 8 ppm. Treated plants are maintained under existing greenhouse conditions for one week. Two bean leaves are removed and placed individually on moist filter paper in Petri dishes. One leaf is infested with 10 third instar larvae of Mexican bean beetle. The other leaf is infested with 10 third instar larvae of Southern armyworm. The dishes are then covered and held for 3 days at which time the percent control (mortality) is determined. A second observation may be made 6 days after infesting the dishes if the experimenter feels the effect may not be complete or moribund insects appear to evidence signs of some recovery. Where necessary, untreated bean leaves are introduced into dishes held for a second observation to preclude insect starvation.

The results of the initial insecticidal evaluations are given in Table II.

Armyworm and bean beetle spray (foliar) results are 96 hour observations. Soil treatment results are 72 hour observations. At the discretion of the experimenter, particular evaluations were held for 144 hour observations. If, after 144 hours, there was a change in the percent control, it is shown in parentheses.

TABLE II

| Biological Evaluations | | | | |
|---|---|---|---|---|
| Example No. | Foliar Application | | Soil Application | |
| | Test Species | | Test Species | |
| | SAW | MBB | MBB | SAW |
| 1 | 0 | 10 | 0 | 0 |
| 2 | 0 | 10 | 0 | 0 |
| 3 | 100 | 0 | - | - |
| 4 | 100 | 20 | - | - |
| 5 | 100 | 20 | - | - |
| 6 | 100 | 10 | - | - |
| 7 | 100 | 0 | - | - |
| 8 | 100 | 40 | - | - |
| 9 | 100 | 20 | - | - |

It should be understood that the instant specification and examples are set forth by way of illustration and not limitation and that various modifications and changes may be made without departing from the spirit and scope of the present invention as defined by the appended claims.

## Claims

**1.** An insecticidally active compound having the formula

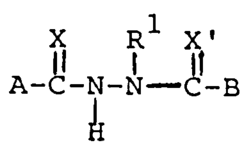

wherein

X and X' — are the same or different O, S or NR;

$R^1$ is — unsubstituted ($C_3$-$C_{10}$)branched alkyl or a ($C_1$-$C_4$) straight chain alkyl substituted with one or two of the same or different ($C_3$-$C_6$) cycloalkyl; and

one of A and B is — unsubstituted naphthyl or substituted naphthyl where the substituents can be from one to three of the same or different halo; cyano; nitro; hydroxy; ($C_1$-$C_4$)alkoxy; ($C_1$-$C_4$)alkyl; carboxy; ($C_1$-$C_4$)alkoxycarbonyl; ($C_1$-$C_4$)alkanoyloxy; amino; ($C_1$-$C_4$)alkylamino or di($C_1$-$C_4$)alkylamino; or

unsubstituted phenyl or substituted phenyl where the substituents can be from one to five of the same or different halo; nitro; cyano; hydroxy; ($C_1$-$C_6$)alkyl; ($C_1$-$C_6$)haloalkyl; cyano($C_1$-$C_6$)alkyl; ($C_1$-$C_6$)-alkoxy; halo($C_1$-$C_6$)alkoxy; ($C_1$-$C_6$)alkoxy($C_1$-$C_6$)alkyl; ($C_1$-$C_6$)alkoxy-($C_1$-$C_6$)alkoxy; carboxyoxy; ($C_1$-$C_6$)alkoxycarbonyloxy($C_2$-$C_6$)alkenyl optionally substituted with halo, cyano, ($C_1$-$C_4$)alkyl, ($C_1$-$C_4$)alkoxy, halo($C_1$-$C_4$)alkoxy or ($C_1$-$C_4$)alkylthio; carboxy; carboxy($C_1$-$C_6$)alkyl; ($C_1$-$C_6$)alkoxycarbonyl($C_1$-$C_6$)alkyl; -COR; halo($C_1$-$C_6$)alkylcarbonyl; cyano($C_1$-$C_6$)alkylcarbonyl; nitro($C_1$-$C_6$)alkylcarbonyl; ($C_1$-$C_6$)-alkoxycarbonyl; halo($C_1$-$C_6$)alkoxycarbonyl; alkanoyloxy; amino; ($C_1$-$C_6$)alkylamino; di($C_1$-$C_6$)alkylamino; amino or ($C_1$-$C_6$)alkylamino where the N of the amino or ($C_1$-$C_6$)alkylamino is substituted with a hydroxy, ($C_1$-$C_4$)alkoxy or ($C_1$-$C_4$)alkylthio group; phenylamino; diphenylamino; -CONRR'; -OCONRR'; -NRCOR'; -$NRCO_2R'$; -N-

(COR)COR'; -OCONRCOR'; sulfhydryl; halothio; $(C_1-C_6)$alkylthio; halo$(C_1-C_6)$alkylthio; $(C_1-C_6)$alkylsulfinyl; $(C_1-C_6)$alkylsulfonyl; phenylsulfonyl; $(C_1-C_6)$alkylsulfonyloxy; halo$(C_1-C_6)$alkylsulfonyloxy; $-SO_2NRR'$; -NRSOR'; $-NRSO_2R'$; -CSR; $-CS_2R$; -NRCSR'; -SCOR; unsubstituted phenyl; phenyl substituted with one to three of the same or different halo, cyano, nitro, hydroxy $(C_1-C_4)$alkyl, $(C_1-C_4)$-alkoxy, carboxy, $(C_1-C_4)$alkoxycarbonyl, $(C_1-C_4)$alkanoyloxy, amino, $(C_1-C_4)$alkylamino or di$(C_1-C_4)$alkylamino; phenoxy where the phenyl ring is unsubstituted or substituted with one to three of the same or different halo, cyano, nitro, hydroxy, $(C_1-C_4)$alkyl, $(C_1-C_4)$alkoxy, carboxy, $(C_1-C_4)$alkoxycarbonyl, $(C_1-C_4)$alkanoyloxy, amino, $(C_1-C_4)$-alkylamino or di$(C_1-C_4)$alkylamino; or phenylthio where the phenyl ring is unsubstituted or substituted with one to three of the same or different halo, cyano, nitro, hydroxy, $(C_1-C_4)$alkyl, $(C_1-C_4)$alkoxy, carboxy, $(C_1-C_4)$alkoxycarbonyl, $(C_1-C_4)$alkanoyloxy, amino, $(C_1-C_4)$-alkylamino or di$(C_1-C_4)$alkylamino; or when two adjacent positions on the phenyl ring are substituted with alkoxy groups, these groups may be joined to form a 5- or 6- membered dioxolano or dioxano heterocyclic ring; and

the other one of A and B is unsubstituted adamantyl or substituted adamantyl having one to four of the same or different halo, cyano, nitro, hydroxy, $(C_{1-4})$ alkyl, halo-$(C_1-C_4)$alkyl, $(C_1-C_4)$ alkoxy, halo$(C_1-C_4)$alkoxy, carboxy, $(C_1-C_4)$-alkanoyl, $C_1-C_4)$alkoxycarbonyl, $(C_{1-4})$alkanoyloxy, amino, $(C_1-C_4)$ alkylamino or di$(C_1-C_4)$alkylamino;

where

R and R' are independently hydrogen or $(C_1-C_6)$alkyl; and agronomically acceptable salts thereof.

**2.** A compound according to Claim 1 wherein

X and X' are O or S;

$R^1$ is unsubstituted $(C_3-C_{10})$branched alkyl or a $(C_1-C_4)$ straight chain alkyl substituted with one or two of the same or different $(C_3-C_4)$cycloalkyl.

A is adamantyl; and

B is unsubstituted naphthyl; or unsubstituted or substituted phenyl where the substituents can be from one to three of the same or different halo; nitro; cyano; $(C_1-C_4)$alkyl; halo$(C_1-C_4)$alkyl; cyano$(C_1-C_4)$-alkyl; $(C_1-C_4)$alkoxy; $(C_1-C_4)$alkoxy$(C_1-C_4)$alkyl; -COZ; carboxy; $(C_1-C_4)$alkoxycarbonyl; $(C_1-C_4)$alkanoyloxy; amino; $(C_1-C_4)$alkylamino; di$(C_1-C_4)$alkylamino; $(C_1-C_4)$alkylthio; $(C_1-C_4)$alkylthiocarbonyl; $(C_1-C_4)$alkylthio(thiocarbonyl); -SCOZ; unsubstituted phenyl; substituted phenyl having one or two of the same or different halo, nitro, $(C_1-C_4)$alkyl, $(C_1-C_4)$alkoxy, carboxy, $(C_1-C_4)$alkoxycarbony1, $(C_1-C_4)$alkanoyloxy, amino, $(C_1-C_4)$-alkylamino or di$(C_1-C_4)$alkylamino; or phenoxy where the phenyl ring is unsubstituted or substituted with one or two of the same or different halo, nitro, $(C_1-C_4)$alkyl, $(C_1-C_4)$-alkoxy, carboxy, $(C_1-C_4)$alkoxycarbonyl, $(C_1-C_4)$alkanoyloxy, amino, $(C_1-C_4)$alkylamino or di$(C_1-C_4)$alkylamino;or when two adjacent positions on the phenyl ring are substituted with alkoxy groups, these groups may be joined to form a 5- or 6-membered dioxoloano or dioxano heterocyclic ring;

where

Z is hydrogen or $(C_1-C_4)$alkyl; and agronomically acceptable salts thereof.

**3.** The compound according to Claim 2 wherein X and X' are 0 or S;

$R^1$ is $(C_3-C_8)$branched alkyl;

A is adamantyl; and

B is unsubstituted naphthyl; or unsubstituted phenyl or substituted phenyl having one to three of the same or different halo; nitro; cyano; $(C_1-C_4)$alkyl; halo$(C_1-C_4)$alkyl; cyano$(C_1-C_4)$alkyl; $(C_1-C_4)$alkoxy; $(C_1-C_4)$-alkoxycarbonyl; $(C_1-C_4)$alkanoyloxy; unsubstituted phenyl or substituted phenyl having one

or two of the same or different halo, nitro, ($C_1$-$C_4$)alkyl, ($C_1$-$C_4$)alkoxy, carboxy, ($C_1$-$C_4$)-alkoxycarbonyl, ($C_1$-$C_4$)alkanoyloxy, amino, ($C_1$-$C_4$)alkylamino or di($C_1$-$C_4$)alkylamino; and agronomically acceptable salts thereof.

**4.** The compound according to Claim 3 wherein X and X' are 0;
$R^1$ is ($C_4$-$C_7$)branched alkyl;
A is adamantyl: and
B is unsubstituted phenyl or substituted phenyl where the substituents can be from one to three of the same or different halo, nitro, ($C_1$-$C_4$)alkyl, ($C_1$-$C_4$)alkoxy or halo($C_1$-$C_4$)alkyl; and agronomically acceptable salts thereof.

**5.** A compound according to Claim 4 wherein
X and X' are 0;
$R^1$ is t-butyl, 2,2-dimethylpropyl or 1,2,2-trimethylpropyl;
A is adamantyl; and
B is unsubstituted phenyl or substituted phenyl where the substituents can be one or two of the same or different chloro, fluoro, bromo, iodo, nitro, methyl, ethyl, methoxy or trifluoromethyl; and
agronomically acceptable salts thereof.

**6.** A compound according to Claim 5 wherein X and X' are 0; $R^1$ is t-butyl;
A is 1-adamantyl;and B is selected from the group consisting of phenyl, 3,5-dimethylphenyl, 2-bromophenyl, 3,5-dichlorophenyl, 2,4-dichlorophenyl, 2-nitrophenyl and 2-chloro-4-fluorophenyl.

**7.** A compound according to Claim 2 wherein B is unsubstituted phenyl, 4-chlorophenyl, 3-substituted phenyl, 2-substituted phenyl or disubstituted phenyl.

**8.** A compound according to Claim 1 wherein X and X' are 0 or S;
$R^1$ is unsubstituted ($C_4$-$C_8$)branched alkyl or a straight chain ($C_1$-$C_4$)alkyl substituted with one or two of the same or different cyclo($C_3$-$C_4$)alkyl;
A is unsubstituted naphthyl; or
unsubstituted phenyl or substituted phenyl where the substituents can be from one to three of the same or different halo; nitro; cyano; ($C_1$-$C_4$)alkyl; halo($C_1$-$C_4$)alkyl; cyano($C_1$-$C_4$)alkyl; ($C_1$-$C_4$)alkoxy; ($C_1$-$C_4$)alkoxy($C_1$-$C_4$)alkyl; -COZ; carboxy; ($C_1$-$C_4$)alkoxycarbonyl; ($C_1$-$C_4$)-alkanoyloxy; amino; ($C_1$-$C_4$)alkylamino; di($C_1$-$C_4$)alkylamino; ($C_1$-$C_4$)alkylthio; ($C_1$-$C_4$)-alkylthiocarbonyl; ($C_1$-$C_4$)alkyldithionate; -SCOZ; unsubstituted phenyl; substituted phenyl having one to two of the same or different halo, nitro, ($C_1$-$C_4$)alkyl, ($C_1$-$C_4$)alkoxy, carboxy, ($C_1$-$C_4$)alkoxycarbonyl, ($C_1$-$C_4$)alkanoyloxy, amino, ($C_1$-$C_4$)alkylamino or di($C_1$-$C_4$)-alkylamino; or phenoxy where the phenyl ring is unsubstituted or subsituted with one or two of the same or different halo, nitro, ($C_1$-$C_4$)alkyl, ($C_1$-$C_4$)alkoxy, carboxy, ($C_1$-$C_4$)-alkoxycarbonyl, ($C_1$-$C_4$)alkanoyloxy, amino, ($C_1$-$C_4$)alkylamino or di($C_1$-$C_4$)alkylamino; or when two adjacent positions on the phenyl ring are substituted with alkoxy groups, these groups may be joined to form a 5- or 6-membered dioxolano or dioxano heterocyclic ring; and
B is unsubstituted adamantyl or substituted adamantyl having one or two of the same or different halo, ($C_1$-$C_4$)alkyl, halo($C_1$-$C_4$)alkyl, ($C_1$-$C_4$)alkoxy, ($C_1$-$C_4$)alkanoyl or halo($C_1$-$C_4$)-alkoxy; and
agronomically acceptable salts thereof.

**9.** A compound according to Claim 8 wherein X and X' are 0 or S;
$R^1$ is ($C_4$-$C_8$) branched alkyl,
A is unsubstituted naphthyl; or
unsubstituted phenyl or substituted phenyl having one to three of the same or different halo; nitro; cyano; ($C_1$-$C_4$)alkyl; halo($C_1$-$C_4$)alkyl; cyano($C_1$-$C_4$)alkyl; ($C_1$-$C_4$)alkoxy; -COZ; ($C_1$-$C_4$)alkoxycarbonyl; ($C_1$-$C_4$)alkanoyloxy; unsubstituted phenyl; or substituted phenyl having one or two of the same or different halo, nitro, ($C_1$-$C_4$)alkyl, ($C_1$-$C_4$)alkoxy, carboxy, ($C_1$-$C_4$)alkoxycarbonyl, ($C_1$-$C_4$)alkanoyloxy, amino, ($C_1$-$C_4$)alkylamino or di($C_1$-$C_4$)-

alkylamino; and

B is unsubstituted adamantyl or substituted adamantyl where the substituent is halo, $(C_1-C_4)$-alkyl, $(C_1-C_4)$alkanoyl, $(C_1-C_4)$alkoxy or halo$(C_1-C_4)$ alkoxy; and agronomically acceptable salts thereof.

**10.** A compound according to Claim 9 wherein X and X' are 0;
$R^1$ is $(C_4-C_7)$ branched alkyl;
A is phenyl;and B is 1-adamantyl.

**11.** A compound according to any one of the preceding Claims wherein $R^1$ is t-butyl.

**12.** A compound according to any one of the preceding claims provided that when $R^1$ is $(C_3-C_{10})$alkyl the alkyl group includes a tertiary carbon atom.

**13.** A compound according to Claim 1 wherein the compound is
N'-t-butyl-N-benzoyl-N'-(1-adamantylcarbonyl)hydrazine;
N'-t-butyl-N-(3-methylbenzoyl)-N'-1-adamantyl)carbonyl)hydrazine;
N'-t-butyl-N'-benzoyl-N-(1-adamantyl-carbonyl) hydrazine;
N'-t-butyl-N'-(3,5-dimethylbenzoyl)-N-(1-adamantylcarbonyl)hydrazine;
N'-t-butyl-N'-(2-bromobenzoyl)-N-(1-adamantylcarbonyl)hydrazine;
N'-t-butyl-N'-(3,5-dichlorobenzoyl)-N-(1-adamantylcarbonyl)hydrazine;
N'-t-butyl-N'-(2,4-dichlorobenzoyl)-N-(1-adamantylcarbonyl)hydrazine;
N'-t-butyl-N'-(2-nitrobenzoyl)-N-(1-adamantylcarbonyl)hydrazine; or
N'-t-butyl-N'-(2-chloro-4-fluorobenzoyl-)N-(1-adamantylcarbonyl)hydrazine.

**14.** A pesticidal composition comprising, as an active ingredient, a compound according to anyone of the preceding Claims, or an agronomically acceptable salt thereof, and an agronomically acceptable diluent or carrier.

**15.** The composition according to Claim 14 wherein the active compound is present at from 0.001 to 90% by weight of the composition, preferably from 0.01 to 75% by weight of the composition.

**16.** The composition according to Claim 14 or Claim 15 wherein said compoisition additionally comprises either (a) a dispersing agent, the composition being in the form of a wettable powder; or (b) a liquid agronomically acceptable carrier and a dispersing agent, the composition being flowable; or (c) a binding agent, the composition being in the form of a granule, or (d) an attractant agent, the composition being in the form of a bait; or (e) an emulsifying agent, the composition being in the form or an emulsifiable concentrate.

**17.** The composition according to Claim 14 or Claim 15 wherein the composition is in the form of a dust.

**18.** A method of combating insect infestation in plants, which method comprises applying an insecticidally active compound as defined in any one of Claims 1 to 13, optionally in a composition as claimed in any one of Claims 14 to 17, to either growing plants, or seeds, or an area where or a medium in which plants are to be grown or are growing.

**19.** A vendible article comprising:
(a) a container and
(b) an insecticidally active compound as claimed in any one of Claims 1 to 12, optionally in a composition as claimed in anyone of Claims 13 to 17.

## Patentansprüche

1. Insektizid wirkende Verbindung der Formel

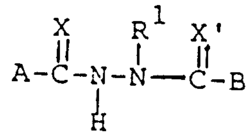

worin

X und X' gleich oder verschieden sind und O, S oder NR bedeuten;

$R^1$ eine unsubstituierte ($C_3$-$C_{10}$)verzweigte Alkylgruppe oder eine ($C_1$-$C_4$)geradkettige Alkylgruppe ist, die mit einem oder zwei gleichen oder verschiedenen ($C_3$-$C_6$)Cycloalkylresten substituiert ist; und

einer der Reste A und B unsubstituiertes Naphthyl oder substituiertes Naphthyl ist, wobei die Substituenten eine bis drei gleiche oder verschiedene der folgenden Gruppen sein können: Halogen; Cyano; Nitro; Hydroxy; ($C_1$-$C_4$)Alkoxy; ($C_1$-$C_4$)Alkyl; Carboxy; ($C_1$-$C_4$)Alkoxycarbonyl; ($C_1$-$C_4$)Alkanoyloxy; Amino; ($C_1$-$C_4$)Alkylamino oder Di($C_1$-$C_4$)-alkylamino oder

unsubstituiertes Phenyl oder substituiertes Phenyl, wobei die Substituenten eine bis fünf gleiche oder verschiedene der folgenden Gruppen sein können: Halogen; Nitro; Cyano; Hydroxy; ($C_1$-$C_6$)-Alkyl; ($C_1$-$C_6$)Halogenalkyl; Cyano($C_1$-$C_6$)alkyl; ($C_1$-$C_6$)Alkoxy; Halogen($C_1$-$C_6$)alkoxy; ($C_1$-$C_6$)Alkoxy($C_1$-$C_6$)alkyl; ($C_1$-$C_6$)Alkoxy-($C_1$-$C_6$)alkoxy; Carboxyoxy; ($C_1$-$C_6$)Alkoxycarbonyloxy($C_2$-$C_6$)-alkenyl; gegebenenfalls substituiert mit Halogen, Cyano, ($C_1$-$C_4$)-Alkyl, ($C_1$-$C_4$)Alkoxy, Halogen($C_1$-$C_4$)alkoxy oder ($C_1$-$C_4$)Alkylthio; Carboxy; Carboxy($C_1$-$C_6$)alkyl; ($C_1$-$C_6$)Alkoxycarbonyl($C_1$-$C_6$)alkyl; -COR; Halogen($C_1$-$C_6$)alkylcarbonyl; Cyano($C_1$-$C_6$)alkylcarbonyl; Nitro($C_1$-$C_6$)alkylcarbonyl; ($C_1$-$C_6$)Alkoxycarbonyl; Halogen($C_1$-$C_6$)-alkoxycarbonyl; Alkanoyloxy; Amino; ($C_1$-$C_6$)Alkylamino; Di($C_1$-$C_6$)alkylamino; Amino oder ($C_1$-$C_6$)Alkylamino, wo das N der Amino- oder ($C_1$-$C_6$)Alkylaminogruppe mit einer Hydroxy-, ($C_1$-$C_4$)Alkoxy- oder ($C_1$-$C_4$)Alkylthiogruppe substituiert ist; Phenylamino; Diphenylamino; -CONRR', -OCONRR', -NRCOR'-, $-NRCO_2R'$, -N(COR)COR'; -OCONRCOR'; Sulfhydryl; Halogenthio; ($C_1$-$C_6$)-Alkylthio; Halogen($C_1$-$C_6$)alkylthio; ($C_1$-$C_6$)Alkylsulfinyl; ($C_1$-$C_6$)-Alkylsulfonyl; Phenylsulfonyl; ($C_1$-$C_6$)Alkylsulfonyloxy; Halogen($C_1$-$C_6$)alkylsulfonyloxy; $-SO_2NRR'$; -NRSOR'; $-NRSO_2R'$; -CSR; $-CS_2R$; -NRCSR'; -SCOR; unsubstituiertes Phenyl; Phenyl, das mit einer bis drei der gleichen oder verschiedenen folgenden Gruppen substituiert ist: Halogen, Cyano, Nitro, Hydroxy, ($C_1$- $C_4$)Alkyl, ($C_1$-$C_4$)Alkoxy, Carboxy, ($C_1$-$C_4$)Alkoxycarbonyl, ($C_1$-$C_4$)Alkanoyloxy, Amino, ($C_1$-$C_4$)Alkylamino oder Di($C_1$-$C_4$)alkylamino;

Phenoxy, worin der Phenylring unsubstituiert ist oder mit einer bis drei der gleichen oder verschiedenen folgenden Gruppen substituiert ist: Halogen, Cyano, Nitro, Hydroxy, ($C_1$-$C_4$)Alkyl, ($C_1$-$C_4$)-Alkoxy, Carboxy, ($C_1$-$C_4$)Alkoxycarbonyl, ($C_1$-$C_4$)Alkanoyloxy, Amino, ($C_1$-$C_4$)Alkylamino oder Di($C_1$-$C_4$)alkylamino; oder

Phenylthio, worin der Phenylring unsubstituiert ist oder mit einer bis drei der gleichen oder verschiedenen folgenden Gruppen substituiert ist: Halogen, Cyano, Nitro, Hydroxy, ($C_1$-$C_4$)Alkyl, ($C_1$-$C_4$)Alkoxy, Carboxy, ($C_1$-$C_4$)Alkoxycarbonyl, ($C_1$-$C_4$)Alkanoyloxy, Amino, ($C_1$-$C_4$)Alkylamino oder Di($C_1$-$C_4$)alkylamino; oder wenn

| | |
|---|---|
| | zwei benachbarte Stellungen im Phenylring mit Alkoxygruppen substituiert sind, können diese Gruppen unter Bildung eines 5- oder 6-gliedrigen Dioxolan- oder Dioxan-Heterozyklusringes verbunden sein und |
| der andere der Reste A und B | ist unsubstituiertes Adamantyl oder substituiertes Adamantyl, das mit einer bis vier der gleichen oder verschiedenen folgenden Gruppen substituiert ist; Halogen, Cyano, Nitro, Hydroxy, $(C_1\text{-}C_4)$-Alkyl, Halogen$(C_1\text{-}C_4)$alkyl, $(C_1\text{-}C_4)$Alkoxy, Halogen$(C_1\text{-}C_4)$alkoxy, Carboxy, $(C_1\text{-}C_4)$Alkanoyl, $(C_1\text{-}C_4)$Alkoxycarbonyl, $(C_1\text{-}C_4)$-Alkanoyloxy, Amino, $(C_1\text{-}C_4)$Alkylamino oder Di$(C_1\text{-}C_4)$alkylamino; wobei |
| R und R' | unabhängig voneinander Wasserstoff oder $(C_1\text{-}C_6)$Alkyl sind, und landwirtschaftlich annehmbare Salze davon. |

**2.** Verbindung nach Anspruch 1, dadurch gekennzeichnet, daß

| | |
|---|---|
| X und X' | O oder S sind, |
| $R^1$ | unsubstituiertes verzweigtes $(C_3\text{-}C_{10})$Alkyl oder ein geradkettiges $(C_1\text{-}C_4)$Alkyl ist, das mit ein bis drei gleichen oder verschiedenen $(C_3\text{-}C_4)$Cycloalkylgruppen substituiert ist. |
| A | Adamantyl ist, und |
| B | unsubstituiertes Naphthyl oder unsubstituiertes oder substituiertes Phenyl ist, worin die Substituenten eine bis drei gleiche oder verschiedene der folgenden Gruppen sein können: Halogen; Nitro; Cyano; $(C_1\text{-}C_4)$Alkyl; Halogen$(C_1\text{-}C_4)$alkyl; Cyano$(C_1\text{-}C_4)$alkyl; $(C_1\text{-}C_4)$Alkoxy; $(C_1\text{-}C_4)$Alkoxy$(C_1\text{-}C_4)$alkyl; -COZ; Carboxy; $(C_1\text{-}C_4)$Alkoxycarbonyl; $(C_1\text{-}C_4)$Alkanoyloxy; Amino; $(C_1\text{-}C_4)$Alkylamino; Di$(C_1\text{-}C_4)$alkylamino; $(C_1\text{-}C_4)$Alkylthio; $(C_1\text{-}C_4)$Alkylthiocarbonyl; $(C_1\text{-}C_4)$Alkylthio(thiocarbonyl); -SCOZ, unsubstituiertes Phenyl, substituiertes Phenyl mit einer oder drei gleichen oder verschiedenen der folgenden Gruppen: Halogen, Nitro, $(C_1\text{-}C_4)$Alkyl, $(C_1\text{-}C_4)$- Alkoxy, Carboxy, $(C_1\text{-}C_4)$-Alkoxycarbonyl, $(C_1\text{-}C_4)$- Alkanoyloxy, Amino, $(C_1\text{-}C_4)$Alkylamino oder Di$(C_1\text{-}C_4)$-alkylamino;<br>oder Phenoxy, worin der Phenylring unsubstituiert ist oder mit einer oder zwei gleichen oder verschiedenen der folgenden Gruppen substituiert ist: Halogen, Nitro, $(C_1\text{-}C_4)$Alkyl, $(C_1\text{-}C_4)$Alkoxy, Carboxy, $(C_1\text{-}C_4)$Alkoxycarbonyl, $(C_1\text{-}C_4)$Alkanoyloxy, Amino, $(C_1\text{-}C_4)$-Alkylamino oder Di$(C_1\text{-}C_4)$alkylamino;<br>oder, wenn zwei benachbarte Stellungen im Phenylring mit Alkoxygruppen substituiert sind, diese Gruppen, unter Bildung eines 5- oder 6-gliedrigen Dioxolan- oder Dioxan-Heterozyklusringes verbunden sein können, |
| Z | Wasserstoff oder $(C_1\text{-}C_4)$Alkyl ist, und<br>landwirtschaftlich annehmbare Salze davon. |

**3.** Verbindung nach Anspruch 2, dadurch gekennzeichnet, daß

| | |
|---|---|
| X und X' | O oder S sind; |
| $R^1$ | verzweigtes $(C_3\text{-}C_8)$Alkyl ist, |
| A | Adamantyl ist, und |
| B | unsubstituiertes Naphthyl oder unsubstituiertes Phenyl oder substituiertes Phenyl ist, das eine bis drei gleiche oder verschiedene der folgenden Gruppen hat: Halogen; Nitro; Cyano; $(C_1\text{-}C_4)$Alkyl; Halogen$(C_1\text{-}C_4)$alkyl; Cyano$(C_1\text{-}C_4)$alkyl; $(C_1\text{-}C_4)$Alkoxy; $(C_1\text{-}C_4)$-Alkoxycarbonyl; $(C_1\text{-}C_4)$Alkanoyloxy; unsubstituiertes Phenyl oder substituiertes Phenyl mit einer oder zwei der folgenden gleichen oder verschiedenen Gruppen: Halogen, Nitro, $(C_1\text{-}C_4)$Alkyl, $(C_1\text{-}C_4)$Alkoxy, Carboxy, $(C_1\text{-}C_4)$Alkoxycarbonyl, $(C_1\text{-}C_4)$-Alkanoyloxy, Amino, $(C_1\text{-}C_4)$Alkylamino oder Di$(C_1\text{-}C_4)$Alkylamino, und<br>landwirtschaftlich annehmbare Salze davon. |

**4.** Verbindung nach Anspruch 3, dadurch gekennzeichnet, daß

| | |
|---|---|
| X und X' | O sind; |
| $R^1$ | verzweigtes $(C_4\text{-}C_7)$Alkyl ist, |
| A | Adamantyl ist, und |
| B | unsubstituiertes Phenyl oder substituiertes Phenyl ist, worin die Substituenten eine bis drei gleiche oder verschiedene der folgenden Gruppen sein können: Halogen, Nitro, |

($C_1$-$C_4$)Alkyl, ($C_1$-$C_4$)Alkoxy oder Halogen($C_1$-$C_4$)alkyl, und
landwirtschaftlich annehmbare Salze davon.

**5.** Verbindung nach Anspruch 4, dadurch gekennzeichnet, daß
X und X' O sind;
$R^1$ t-Butyl, 2,2-Dimethylpropyl oder 1,2,2-Trimethylpropyl ist,
A Adamantyl ist, und
B unsubstituiertes Phenyl oder substituiertes Phenyl ist, worin die Substituenten eine oder zwei gleiche oder verschiedene der folgenden Gruppen sein können: Chlor, Fluor, Brom, Jod, Nitro, Methyl, Ethyl, Methoxy oder Trifluormethyl, und
landwirtschaftlich annehmbare Salze davon.

**6.** Verbindung nach Anspruch 5, dadurch gekennzeichnet, daß
X und X' O sind,
$R^1$ t-Butyl ist,
A 1-Adamantyl ist, und
B aus der Gruppe Phenyl, 3,5-Dimethylphenyl, 2-Bromphenyl, 3,5-Dichlorphenyl, 2,4-Dichlorphenyl, 2-Nitrophenyl und 2-Chlor-4-fluorphenyl gewählt ist.

**7.** Verbindung nach Anspruch 2, dadurch gekennzeichnet, daß B unsubstituiertes Phenyl, 4-Chlorphenyl, 3-substituiertes Phenyl, 2-substituiertes Phenyl oder disubstituiertes Phenyl ist.

**8.** Verbindung nach Anspruch 1, dadurch gekennzeichnet, daß X und X' O oder S sind,
$R^1$ unsubstituiertes verzweigtes ($C_4$-$C_8$)Alkyl oder ein geradkettiges ($C_1$-$C_4$)Alkyl ist, das mit einer oder zwei gleichen oder verschiedenen Cyclo($C_3$-$C_4$)alkylgruppen substituiert ist,
A unsubstituiertes Naphthyl ist
oder unsubstituiertes Phenyl oder substituiertes Phenyl, worin die Substituenten eine bis drei gleiche oder verschiedene folgende Gruppen sein können:
Halogen; Nitro; Cyano; ($C_1$-$C_4$)Alkyl; Halogen($C_1$-$C_4$)alkyl; Cyano($C_1$-$C_4$)alkyl; ($C_1$-$C_4$)Alkoxy; ($C_1$-$C_4$)Alkoxy($C_1$-$C_4$)alkyl; -COZ; Carboxy; ($C_1$-$C_4$)Alkoxycarbonyl; ($C_1$-$C_4$)Alkanoyloxy; Amino; ($C_1$-$C_4$)Alkylamino; Di($C_1$-$C_4$)Alkylamino; ($C_1$-$C_4$)Alkylthio; ($C_1$-$C_4$)Alkylthiocarbonyl; ($C_1$-$C_4$)Alkyldithionat; -SCOZ; unsubstituiertes Phenyl, substituiertes Phenyl mit einer oder zwei gleichen oder verschiedenen der folgenden Gruppen: Halogen, Nitro, ($C_1$-$C_4$)Alkyl, ($C_1$-$C_4$)-Alkoxy, Carboxy, ($C_1$-$C_4$)Alkoxycarbonyl, ($C_1$-$C_4$)Alkanoyloxy, Amino, ($C_1$-$C_4$)Alkylamino oder Di($C_1$-$C_4$)alkylamino,
oder Phenoxy, worin der Phenylring unsubstituiert ist oder mit einer oder zwei gleichen oder verschiedenen der folgenden Gruppen substituiert ist: Halogen, Nitro, ($C_1$-$C_4$)Alkyl, ($C_1$-$C_4$)-Alkoxy, Carboxy, ($C_1$-$C_4$)Alkoxycarbonyl, ($C_1$-$C_4$)Alkanoyloxy, Amino, ($C_1$-$C_4$)Alkylamino oder Di($C_1$-$C_4$)alkylamino; oder
wenn zwei benachbarte Stellungen am Phenylring mit Alkoxygruppen substituiert sind, diese Gruppen unter Bildung eines 5- oder 6-gliedrigen Dioxolan- oder Dioxan-Heterozyklusringes verbunden sein können, und
B unsubstituiertes Adamantyl oder substituiertes Adamantyl ist, das eine oder zwei gleiche oder verschiedene der folgende Gruppen hat: Halogen, ($C_1$-$C_4$)Alkyl, Halogen($C_1$-$C_4$)alkyl, ($C_1$-$C_4$)-Alkoxy, ($C_1$-$C_4$)Alkanoyl oder Halogen($C_1$-$C_4$)alkoxy; und
landwirtschaftlich annehmbare Salze davon.

**9.** Verbindung nach Anspruch 8, dadurch gekennzeichnet, daß
X und X' O oder S sind,
$R^1$ verzweigtes ($C_4$-$C_8$)alkyl ist,
A unsubstituiertes Naphthyl oder unsubstituiertes Phenyl oder substituiertes Phenyl ist, das eine bis drei gleiche oder verschiedene der folgenden Gruppen hat: Halogen; Nitro; Cyano; ($C_1$-$C_4$)Alkyl; Halogen($C_1$-$C_4$)alkyl; Cyano($C_1$-$C_4$)alkyl; ($C_1$-$C_4$)Alkoxy; -COZ; ($C_1$-$C_4$)Alkoxycarbonyl; ($C_1$-$C_4$)Alkanoyloxy; unsubstituiertes Phenyl oder substituiertes Phenyl mit einer oder zwei gleichen oder verschiedenen der folgenden Gruppen: Halogen, Nitro, ($C_1$-$C_4$)Alkyl, ($C_1$-$C_4$)Alkoxy, Carboxy, ($C_1$-$C_4$)Alkoxycarbonyl, ($C_1$-$C_4$)-Alkanoyloxy, Amino, ($C_1$-$C_4$)Alkylamino oder Di($C_1$-$C_4$)alkylamino, und

B unsubstituiertes Adamantyl oder substituiertes Adamantyl ist, worin der Substituent Halogen, $(C_1\text{-}C_4)$Alkyl, $(C_1\text{-}C_4)$Alkanoyl, $(C_1\text{-}C_4)$Alkoxy oder Halogen$(C_1\text{-}C_4)$alkoxy ist, und
landwirtschaftlich annehmbare Salze davon.

**10.** Verbindung nach Anspruch 9, dadurch gekennzeichnet, daß
X und X' O sind:
$R^1$ verzweigtes $(C_4\text{-}C_7)$Alkyl ist,
A Phenyl ist; und
B 1-Adamantyl ist.

**11.** Verbindung nach einem der vorhergehenden Ansprüche, dadurch gekennzeichnet, daß $R^1$ t-Butyl ist.

**12.** Verbindung nach einem der vorhergehenden Ansprüche, dadurch gekennzeichnet, daß dann, wenn $R^1$ $(C_3\text{-}C_{10})$Alkyl ist, die Alkylgruppe ein tertiäres Kohlenstoffatom enthält.

**13.** Verbindung nach Anspruch 1, dadurch gekennzeichnet, daß die Verbindung eine der folgenden ist:
N'-t-Butyl-N-benzoyl-N'-(1-adamantylcarbonyl)hydrazin;
N'-t-Butyl-N-(3-methylbenzoyl)-N'-1-adamantyl-carbonyl)hydrazin;
N'-t-Butyl-N'-benzoyl-N-(1-adamantylcarbonyl)hydrazin;
N'-t-Butyl-N'-(3,5-dimethylbenzoyl)-N-(1-adamantylcarbonyl)hydrazin;
N'-t-Butyl-N'-(2-brombenzoyl)-N-(1-adamantylcarbonyl)hydrazin;
N'-t-Butyl-N'-(3,5-dichlorbenzoyl)-N-(1-adamantylcarbonyl)hydrazin;
N'-t-Butyl-N'-(2,4-dichlorophenyl)-N-(1-adamantylcarbonyl)hydrazin;
N'-t-Butyl-N'-(2-nitrobenzoyl)-N-(1-adamantylcarbonyl)hydrazin; oder
N'-t-Butyl-N'-(2-chlor-4-fluorbenzoyl)-N-(1-adamantylcarbonyl)hydrazin.

**14.** Pestizide Zusammensetzung enthaltend als Wirksubstanz eine Verbindung nach einem der vorhergehenden Ansprüche oder ein landwirtschaftich annehmbares Salz davon und ein landwirtschaftlich annehmbares Verdünnungsmittel oder einen solchen Träger.

**15.** Zusammensetzung nach Anspruch 14, dadurch gekennzeichnet, daß die Wirksubstanz in einer Menge von 0,001 bis 90 Gew.-% der Zusammensetzung vorliegt, vorzugsweise von 0,01 bis 75 Gew.-% der Zusammensetzung.

**16.** Zusammensetzung nach Anspruch 14 oder 15, dadurch gekennzeichnet, daß die Zusammensetzung zusätzlich entweder (a) ein Dispergiermittel enthält, wobei die Zusammensetzung in Form eines benetzbaren Pulvers vorliegt; oder (b) einen flüssigen landwirtschaftlich annehmbaren Träger und ein Dispergiermittel, wobei die Zusammensetzung fließfähig ist oder (c) ein Bindemittel, wobei die Zusammensetzung in Form von Granulat vorliegt oder (d) ein Lockmittel, wobei die Zusammensetzung in Form eines Köders vorliegt oder (e) ein emulgierendes Mittel, wobei die Zusammensetzung in Form eines emulgierbaren Konzentrats vorliegt.

**17.** Zusammensetzung nach Anspruch 14 oder 15, dadurch gekennzeichnet, daß die Zusammensetzung in Form eines Staubs vorliegt.

**18.** Verfahren zur Bekämpfung von Insektenbefall bei Pflanzen, dadurch gekennzeichnet, daß man eine insektizid wirksame Verbindung gemäß einem der Ansprüche 1 bis 12, gegebenenfalls in einer Zusammensetzung nach einem der Ansprüche 13 bis 17, entweder bei wachsenden Pflanzen oder bei Saatgut oder auf eine Fläche wo oder ein Medium in dem Pflanzen wachsen sollen oder wachsen, anwendet.

**19.** Verkäufliches Erzeugnis enthaltend :
(a) einen Behälter, und
(b) eine insektizid wirkende Verbindung nach einem der Ansprüche 1 bis 12, gegebenenfalls in einer Zusammensetzung nach einem der Ansprüche 13 bis 17.

## Revendications

**1.** Composé à action insecticide, possédant la formule

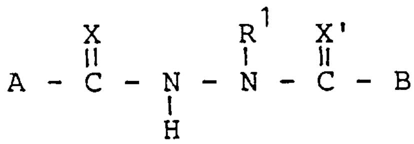

(I)

dans laquelle

X et X' — sont identiques ou différents, et représentent O, S ou NR;

$R^1$ — est un radical alkyle en $C_3$-$C_{10}$ ramifié non substitué ou un radical alkyle en $C_1$-$C_4$ à chaîne droite substitué par un ou deux groupes cycloalkyle en $C_3$-$C_6$ identiques ou différents;

l'un des radicaux A et B — est le radical naphtyle non substitué ou un radical naphtyle substitué par un à trois substituants, identiques ou différents, choisis parmi des atomes d'halogène et des groupes cyano; nitro; hydroxy; alcoxy en $C_1$-$C_4$; alkyle en $C_1$-$C_4$; carboxy; alcoxy($C_1$-$C_4$)-carbonyle; alcanoyloxy en $C_1$-$C_4$; amino; alkyl($C_1$-$C_4$)-amino ou dialkyl($C_1$-$C_4$)-amino; ou le radical phényle non substitué ou un radical phényle substitué par un à cinq substituants, identiques ou différents, choisis parmi des atomes d'halogène et des groupes nitro; cyano; hydroxy; alkyle en $C_1$-$C_6$; halogénoalkyle en $C_1$-$C_6$; cyanoalkyle en $C_1$-$C_6$; alcoxy en $C_1$-$C_6$; halogénoalcoxy en $C_1$-$C_6$; alcoxy($C_1$-$C_6$)-alkyle($C_1$-$C_6$); alcoxy-($C_1$-$C_6$)-alcoxy($C_1$-$C_6$); carboxyoxy; alcoxy($C_1$-$C_6$)-carbonyloxy-alcényle($C_2$-$C_6$) éventuellement substitué par un atome d'halogène ou par un groupe cyano, alkyle en $C_1$-$C_4$, alcoxy en $C_1$-$C_4$, halogénoalcoxy en $C_1$-$C_4$ ou alkyl($C_1$-$C_4$)-thio; carboxy; carboxy-alkyle($C_1$-$C_6$); alcoxy($C_1$-$C_6$)-carbonyl-alkyle($C_1$-$C_6$); -COR; halogénoalkyl($C_1$-$C_6$)-carbonyle; cyanoalkyl($C_1$-$C_6$)-carbonyle; nitroalkyl($C_1$-$C_6$)-carbonyle; alcoxy($C_1$-$C_6$)-carbonyle; halogénoalcoxy($C_1$-$C_6$)carbonyle; alcanoyloxy; amino; alkyl($C_1$-$C_6$)amino; dialkyl($C_1$-$C_6$)-amino; amino ou alkyl-($C_1$-$C_6$)-amino dans lequel l'atome d'azote du groupe amino ou alkyl-($C_1$-$C_6$)-amino est substitué par un groupe hydroxy, alcoxy en $C_1$-$C_4$ ou alkyl-($C_1$-$C_4$)- thio; phénylamino; diphénylamino; -CONRR'; -OCONRR'; -NRCOR'; -$NRCO_2R'$; -N(COR)COR'; -OCONRCOR'; sulfhydryle; halogénothio; alkyl($C_1$-$C_6$)-thio; halogénoalkyl($C_1$-$C_6$)-thio; alkyl($C_1$-$C_6$)-sulfinyle; alkyl($C_1$-$C_6$)-sulfonyle; phénylsulfonyle; alkyl-($C_1$-$C_6$)-sulfonyloxy; halogénoalkyl($C_1$-$C_6$)sulfonyloxy; -$SO_2NRR'$; -NRSOR'; -$NRSO_2R'$; -CSR; -$CS_2R$; -NRCSR'; -SCOR; le groupe phényle non substitué; un groupe phényle substitué par un à trois substituants, identiques ou différents, choisis parmi des atomes d'halogène et des groupes cyano, nitro, hydroxyalkyle en $C_1$-$C_4$, alcoxy en $C_1$-$C_4$, carboxy, alcoxy($C_1$-$C_4$)-carbonyle, alcanoyloxy en $C_1$-$C_4$, amino, alkyl($C_1$-$C_4$)amino ou dialkyl($C_1$-$C_4$)-amino; un groupe phénoxy dans lequel le noyau phényle est non substitué ou substitué par un à trois substituants, identiques ou différents, choisis parmi des atomes d'halogène et des groupes cyano, nitro, hydroxy, alkyle en $C_1$-$C_4$, alcoxy en $C_1$-$C_4$, carboxy, alcoxy($C_1$-$C_4$)-carbonyle, alcanoyloxy en $C_1$-$C_4$, amino, alkyl($C_1$-$C_4$)amino ou dialkyl($C_1$-$C_4$)-amino; ou un groupe phénylthio dans lequel le noyau phényle est non substitué ou substitué par un à trois substituants, identiques ou différents, choisis parmi des atomes d'halogène et des groupes cyano, nitro, hydroxy, alkyle en $C_1$-$C_4$, alcoxy en $C_1$-$C_4$, carboxy, alcoxy($C_1$-$C_4$)-carbonyle, alcanoyloxy en $C_1$-$C_4$, amino, alkyl($C_1$-$C_4$)-amino ou dialkyl($C_1$-$C_4$)-amino; ou, lorsque deux positions adjacentes sur le noyau phényle sont substituées par des groupes alcoxy, ces groupes

peuvent être liés pour former un cycle hétérocyclique dioxolanno ou dioxanno à 5 ou 6 chaînons; et

l'autre des radicaux A et B est le radical adamantyle non substitué ou un radical adamantyle substitué par un à quatre substituants, identiques ou différents, choisis parmi des atomes d'halogène et des groupes cyano, nitro, hydroxy, alkyle en $C_1$-$C_4$, halogénoalkyle en $C_1$-$C_4$, alcoxy en $C_1$-$C_4$, halogénoalcoxy en $C_1$-$C_4$, carboxy, alcanoyle en $C_1$-$C_4$, alcoxy($C_1$-$C_4$)-carbonyle, alcanoyloxy en $C_1$-$C_4$, amino, alkyl($C_1$-$C_4$)-amino ou dialkyl($C_1$-$C_4$)-amino;

R et R' étant indépendamment un atome d'hydrogène ou un groupe alkyle en $C_1$-$C_6$; et sels agronomiquement acceptables de celui-ci.

**2.** Composé selon la revendication 1, dans lequel

X et X' sont O ou S;

$R^1$ est un radical alkyle en $C_3$-$C_{10}$ ramifié non substitué ou un radical alkyle en $C_1$-$C_4$ à chaîne droite substitué par un ou deux groupes cycloalkyle en $C_3$-$C_4$ identiques ou différents;

A est le radical adamantyle; et

B est le radical naphtyle non substitué; ou le radical phényle non substitué ou un radical phényle substitué par un à trois substituants, identiques ou différents, choisis parmi des atomes d'halogène et des groupes nitro; cyano; alkyle en $C_1$-$C_4$; halogénoalkyle en $C_1$-$C_4$; cyanoalkyle en $C_1$-$C_4$; alcoxy en $C_1$-$C_4$; alcoxy-($C_1$-$C_4$)-alkyle($C_1$-$C_4$); -COZ; carboxy; alcoxy($C_1$-$C_4$)-carbonyle; alcanoyloxy en $C_1$-$C_4$; amino; alkyl($C_1$-$C_4$)-amino; dialkyl($C_1$-$C_4$)-amino; alkyl($C_1$-$C_4$)-thio; alkyl-($C_1$-$C_4$)-thiocarbonyle; alkyl($C_1$-$C_4$)-thio(thiocarbonyle); -SCOZ; le groupe phényle non substitué; un groupe phényle substitué par un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, amino, alkyl-($C_1$-$C_4$)amino ou dialkyl($C_1$-$C_4$)-amino; ou un groupe phénoxy dans lequel le noyau phényle est non substitué ou substitué par un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, amino, alkyl-($C_1$-$C_4$)amino ou dialkyl($C_1$-$C_4$)-amino; ou, lorsque deux positions adjacentes sur le noyau phényle sont substituées par des groupes alcoxy, ces groupes peuvent être liés pour former un cycle hétérocyclique dioxolanno ou dioxanno à 5 ou 6 chaînons;

Z étant un atome d'hydrogène ou un groupe alkyle en $C_1$-$C_4$; et sels agronomiquement acceptables de celui-ci.

**3.** Composé selon la revendication 2, dans lequel

X et X' sont O ou S;

$R^1$ est un groupe alkyle en $C_3$-$C_8$ ramifié;

A est le groupe adamantyle; et

B est le radical naphtyle non substitué ou un radical phényle on substitué ou substitué par un à trois substituants, identiques ou différents, choisis parmi des atomes d'halogène et des groupes nitro; cyano; alkyle en $C_1$-$C_4$; halogénoalkyle en $C_1$-$C_4$; cyanoalkyle en $C_1$-$C_4$; alcoxy en $C_1$-$C_4$; alcoxy-($C_1$-$C_4$)-carbonyle; alcanoyloxy en $C_1$-$C_4$; le groupe phényle non substitué; un groupe phényle substitué par un ou deux substituants, identiques ou différents, choisis parmi des atomes d'halogène et des groupes nitro, alkyle en $C_1$-$C_4$,alcoxy en $C_1$-$C_4$, carboxy, alcoxy($C_1$-$C_4$)-carbonyle, alcanoyloxy en $C_1$-$C_4$, amino, alkyl($C_1$-$C_4$)amino ou dialkyl-($C_1$-$C_4$)-amino; et sels agronomiquement acceptables de celui-ci.

**4.** Composé selon la revendication 3, dans lequel

X et X' sont O;

$R^1$ est un groupe alkyle en $C_4$-$C_7$ ramifié;

A est le groupe adamantyle; et

B est le radical phényle non substitué ou un radical phényle substitué par un à trois substituants, identiques ou différents, choisis parmi des atomes d'halogène et des groupes nitro; alkyle en $C_1$-$C_4$; alcoxy en $C_1$-$C_4$ ou halogénoalkyle en $C_1$-$C_4$; et sels agronomiquement acceptables de celui-ci.

**5.** Composé selon la revendication 4, dans lequel
X et X' sont O;
$R^1$ est le groupe tert-butyle, 2,2-diméthylpropyle ou 1,2,2-triméthylpropyle;
A est le groupe adamantyle; et
B est le radical phényle non substitué ou un radical phényle substitué par un ou deux substituants, identiques ou différents, choisis parmi les atomes de chlore, fluor, brome et iode et les groupes nitro; méthyle, éthyle, méthoxy et trifluorométhyle;
et sels agronomiquement acceptables de celui-ci.

**6.** Composé selon la revendication 5, dans lequel
X et X' sont O;
$R^1$ est le groupe tert-butyle;
A est le groupe 1-adamantyle; et
B est choisi parmi le radical phényle, 3,5-diméthylphényle, 2-bromophényle, 3,5-dichlorophényle, 2,4-dichlorophényle, 2-nitrophényle et 2-chloro-4-fluorophényle.

**7.** Composé selon la revendication 2, dans lequel B est le radical phényle non substitué ou un radical 4-chlorophényle, phényle substitué en position 3, phényle substitué en position 2 ou phényle disubstitué.

**8.** Composé selon la revendication 1, dans lequel
X et X' sont O ou S;
$R^1$ est un radical alkyle en $C_4$-$C_8$ ramifié non substitué ou un radical alkyle en $C_1$-$C_4$ à chaîne droite substitué par un ou deux groupes cycloalkyle en $C_3$-$C_4$ identiques ou différents;
A est le radical naphtyle non substitué; ou
le radical phényle non substitué ou un radical phényle substitué par un à trois substituants, identiques ou différents, choisis parmi des atomes d'halogène et des groupes nitro; cyano; alkyle en $C_1$-$C_4$; halogénoalkyle en $C_1$-$C_4$; cyanoalkyle en $C_1$-$C_4$; alcoxy en $C_1$-$C_4$; alcoxy-($C_1$-$C_4$)-alkyle($C_1$-$C_4$); -COZ; carboxy; alcoxy($C_1$-$C_4$)-carbonyle; alcanoyloxy en $C_1$-$C_4$; amino; alkyl($C_1$-$C_4$)amino; dialkyl($C_1$-$C_4$)-amino; alkyl($C_1$-$C_4$)-thio; alkyl($C_1$-$C_4$)-thiocarbonyle; alkyl($C_1$-$C_4$)-dithionate; -SCOZ; le groupe phényle non substitué; un groupe phényle substitué par un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, amino, alkyl($C_1$-$C_4$)amino ou dialkyl($C_1$-$C_4$)-amino; ou un groupe phénoxy dans lequel le noyau phényle est non substitué ou substitué par un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, amino, alkyl($C_1$-$C_4$)amino ou dialkyl($C_1$-$C_4$)-amino; ou, lorsque deux positions adjacentes sur le noyau phényle sont substituées par des groupes alcoxy, ces groupes peuvent être liés pour former un cycle hétérocyclique dioxolanno ou dioxanno à 5 ou 6 chaînons; et
B est le radical adamantyle non substitué ou un radical adamantyle substitué par un ou deux substituants, identiques ou différents, choisis parmi des atomes d'halogène et des groupes alkyle en $C_1$-$C_4$, halogénoalkyle en $C_1$-$C_4$, alcoxy en $C_1$-$C_4$, alcanoyle en $C_1$-$C_4$ ou halogénoalcoxy en $C_1$-$C_4$;
et sels agronomiquement acceptables de celui-ci.

**9.** Composé selon la revendication 8, dans lequel
X et X' sont O ou S;
$R^1$ est un radical alkyle en $C_4$-$C_8$ ramifié;
A est le radical naphtyle non substitué; ou le radical phényle non substitué ou un radical phényle substitué par un à trois substituants, identiques ou différents, choisis parmi des atomes d'halogène et des groupes nitro; cyano; alkyle en $C_1$-$C_4$; halogénoalkyle en $C_1$-

$C_4$; cyanoalkyle en $C_1$-$C_4$; alcoxy en $C_1$-$C_4$; -COZ; alcoxy($C_1$-$C_4$)-carbonyle; alcanoyloxy en $C_1$-$C_4$; le groupe phényle non substitué; un groupe phényle substitué par un ou deux substituants, identiques ou différents, choisis parmi des atomes d'halogène et des groupes nitro, alkyle en $C_1$-$C_4$, alcoxy en $C_1$-$C_4$, carboxy, alcoxy($C_1$-$C_4$)-carbonyle, alcanoyloxy en $C_1$-$C_4$, amino, alkyl($C_1$-$C_4$)amino ou dialkyl($C_1$-$C_4$)-amino; et

B est le radical adamantyle non substitué ou un radical adamantyle substitué par un atome d'halogène ou un groupe alkyle en $C_1$-$C_4$, alcanoyle en $C_1$-$C_4$, alcoxy en $C_1$-$C_4$ ou halogénoalcoxy en $C_1$-$C_4$;

et sels agronomiquement acceptables de celui-ci.

**10.** Composé selon la revendication 9, dans lequel

X et X' sont O;
$R^1$ est un radical alkyle en $C_4$-$C_7$ ramifié;
A est le radical phényle; et
B est le radical 1-adamantyle.

**11.** Composé selon l'une quelconque des revendications précédentes, dans lequel $R^1$ est le groupe tert-butyle.

**12.** Composé selon l'une quelconque des revendications précédentes, avec la condition que lorsque $R^1$ est un radical alkyle en $C_3$-$C_{10}$, le radical alkyle comprend un atome de carbone tertiaire.

**13.** Composé selon la revendication 1, le composé étant
la N'-tert-butyl-N-benzoyl-N'-(1-adamantylcarbonyl)hydrazine,
la N'-tert-butyl-N-(3-méthylbenzoyl)-N'-(1-adamantylcarbonyl)hydrazine,
la N'-tert-butyl-N'-benzoyl -N-(1-adamantylcarbonyl)hydrazine,
la N'-tert-butyl-N'-(3,5-diméthylbenzoyl)-N-(1-adamantylcarbonyl)hydrazine,
la N'-tert-butyl-N'-(2-bromobenzoyl)-N-(1-adamantylcarbonyl)hydrazine,
la N'-tert-butyl-N'-(3,5-dichlorobenzoyl)-N-(1-adamantylcarbonyl)hydrazine,
la N'-tert-butyl-N'-(2,4-dichlorobenzoyl)-N-(1-adamantylcarbonyl)hydrazine,
la N'-tert-butyl-N'-(2-nitrobenzoyl)-N-(1-adamantylcarbonyl)hydrazine ou
la N'-tert-butyl-N'-(2-chloro-4-fluorobenzoyl)-N-(1-adamantylcarbonyl)hydrazine.

**14.** Composition pesticide comprenant, en tant que composant actif, un composé selon l'une quelconque des revendications précédentes, ou un sel agronomiquement acceptable de celui-ci, et un support ou diluant agronomiquement acceptable.

**15.** Composition selon la revendication 14, dans laquelle le composé actif est présent à raison de 0,001 à 90 % en poids de la composition, de préférence de 0,01 à 75 % en poids de la composition.

**16.** Composition selon la revendication 14 ou 15, dans laquelle ladite composition comprend en outre soit (a) un dispersant, la composition étant sous la forme d'une poudre mouillable; soit (b) un support liquide agronomiquement acceptable et un dispersant, la composition étant apte à l'écoulement; ou (c) un liant, la composition étant sous la forme d'un granule, ou (d) un agent attractif, la composition étant sous la forme d'un appât; ou (e) un émulsifiant, la composition étant sous la forme d'un concentré émulsifiable.

**17.** Composition selon la revendication 14 ou 15, la composition étant sous la forme d'une poudre.

**18.** Procédé de lutte contre l'infestation de plantes par des insectes, lequel procédé comprend l'application d'un composé à action insecticide, tel que défini dans l'une quelconque des revendications 1 à 12, éventuellement dans une composition selon l'une quelconque des revendications 13 à 17, soit sur des plantes en croissance, soit sur des graines, ou dans une zone ou un milieu dans lesquels des plantes se développent ou sont destinées à se développer.

**19.** Article commercialisable, comprenant:
(a) un contenant et

(b) un composé à action insecticide selon l'une quelconque des revendications 1 à 12, éventuellement dans une composition selon l'une quelconque des 13 à 17.